(19) Europäisches Patentamt
European Patent Office
Office européen des brevets

(11) EP 1 046 652 A1

(12) EUROPEAN PATENT APPLICATION
published in accordance with Art. 158(3) EPC

(43) Date of publication:
25.10.2000 Bulletin 2000/43

(21) Application number: 98961418.5

(22) Date of filing: 18.12.1998

(51) Int. Cl.[7]: C07K 19/00, C07K 14/705, C12N 15/62, C07K 17/00, G01N 33/53, A61K 38/17

(86) International application number:
PCT/JP98/05744

(87) International publication number:
WO 99/32520 (01.07.1999 Gazette 1999/26)

(84) Designated Contracting States:
AT BE CH CY DE DK ES FI FR GB GR IE IT LI LU MC NL PT SE

(30) Priority: 19.12.1997 JP 36498197
09.12.1998 JP 34964898
16.12.1998 JP 35817098

(71) Applicant: Japan Tobacco Inc.
Tokyo 105-8422 (JP)

(72) Inventors:
- SAWAMURA, Tatsuya
Kyoto-shi, Kyoto 606-8224 (JP)
- KAKUTANI, Makoto,
Cent. Pharm. Res. Inst. of Japan
Takatsuki-shi, Osaka 569-1125 (JP)
- MASAKI, Tomoh
Kyoto-shi, Kyoto 606-0024 (JP)

(74) Representative:
VOSSIUS & PARTNER
Siebertstrasse 4
81675 München (DE)

# (54) METHOD FOR QUANTITATING DENATURED LDLS

(57) An assay method that can be commonly used in the clinical field whereby denatured LDLs such as oxidized LDL contained in the body fluids of patients with arteriosclerosis, hyperlipidemia, and such can be conveniently detected and quantified in an intact state at a high sensitivity is provided. Also provided is a fusion polypeptide used as a component in the above assay method, which is composed of the extracelluler domain of a mammalian oxidized LDL receptor and a portion of a mammalian immunoglobulin heavy chain. This fusion polypeptide per se is useful as the active ingredient in drugs for preventing and treating arteriosclerosis or hyperlipidemia.

Fig. 2

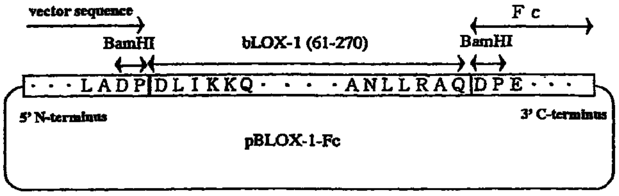


EP 1 046 652 A1

## Description

### Technical field

The present invention relates to a fusion polypeptide composed of an extracellular domain of a mammalian oxidized-LDL receptor and a part of a heavy chain in a mammalian immunoglobulin (Ig), a DNA encoding the fusion polypeptide, a vector comprising the DNA, a transformant transfected by the vector, a fusion polypeptide-immobilized insoluble carrier prepared by immobilizing the fusion polypeptide, a kit used for detecting, quantifying, separating, or purifying an oxidized LDL, a method for detecting, quantifying, separating or purifying an oxidizing LDL, and a pharmaceutical composition comprising the fusion polypeptide.

### Background Art

Cholesterols are present in various forms (free, long-chain fatty acids, or esterified forms) in blood and various tissues. These cholesterols are mainly biosynthesized in the liver. Free cholesterols synthesized in the liver are incorporated into very low density lipoproteins (VLDL), and metabolized via intermediary density lipoproteins (IDL) to low density lipoproteins by the action of lipoprotein lipase (LPL) in the blood and hepatic triglyceride lipase (HTGL). LDL thus formed is taken up into peripheral cells via LDL receptors and plays a critical role in cell membrane construction.

However, LDL is oxidatively denatured by various factors, such as the effect of vascular endothelial cells, various chemical/physical factors, heat and so on to generate denatured LDL called oxidized LDL in blood.

Normally, oxidized LDL is not easily generated in blood due to the sufficient amount of antioxidant substances present in blood stream. Even if generated, most of them are metabolized in the liver.

On the other hand, at the vascular endothelium and vessel walls, oxidized LDL is generated by cell-dependent (vascular endothelial cells, macrophages, and such) chemical denaturation and cell-independent chemical denaturation due to the effect of $Fe^{3+}$ etc. The oxidized LDL generated at the vascular endothelium and vessel walls accumulates in macrophages, which differs from the production in blood.

Oxidized LDL accumulates in macrophages by being taken up into the cells via scavenger receptors on the cell surface of macrophages, which are receptors for various modified LDLs (oxidized LDL, acetylated LDL, succinylated LDL, malondialdehyde LDL) (Nature, 343, 531-535, 1990; ibid., 343, 570-572, 1990; Proc. Natl. Acad. Sci. USA, 87, 9133-9137, 1990; ibid., 87, 8810-8814, 1990: Curr. Opin. Lipodol., 2, 295-300, 1991, and J. Clin. Invest., **90**, 1450-1457, 1992).

Since macrophage scavenger receptors, unlike LDL receptors, do not undergo downregulation occurred depending upon an amount of intracellular cholesterols, macrophages in the subendothelium and inner wall of blood vessels take up a large amount of denatured LDLs and accumulate cholesterols in excess, converting macrophages into foam cells (Chapter IV "Inflammatory cells, 1. Scavenger Receptor" in "Molecular Arteriosclerotic Study", Medical Review, p. 249-258, 1995).

Macrophages creeping into the endothelium or vessel walls described above are those migrated from the blood stream, responding to signals of oxidized LDL synthesis generated at various sites such blood, endothelium or vessel walls, etc. This is due to oxidized LDL which shows chemotaxis for macrophages or monocytes in blood stream, assembling macrophages or monocytes to vascular endothelial cells, inducing the assembled monocytes or macrophages to creep into the vascular endothelium and to be uptaken into vessel walls, inducing the differentiation of the uptaken monocytes into macrophages, and inhibiting the migration of the differentiated macrophages.

It has been being revealed that the oxidized-LDL receptor expressed on vascular endothelial cells, recently identified by the present inventors (designated Oxidized-LDL Receptor, Ox-LDL Receptor or LOX-1, Nature, Vol. 386: 73-77, 1997 and Shishitsu Seikagaku Kenkyu (Lipid Biochemical Study), vol. 39, 83-84, 1997), is closely involved in the assembling of monocytes and macrophages into the vascular endothelial cells.

From previous studies, it has been experimentally demonstrated that production of nitric oxide (NO) in a cell is inhibited by the uptake of oxidized LDL in blood into the vascular endothelium through the oxidized-LDL receptor, inducing, the expression of cell-adhesion molecules on the surface of vascular endothelial cells. From these observations, it has been proposed that macrophages or monocytes are trapped on vascular endothelial cells as a result of the expression of cell-adhesion molecules and the trapped macrophages and monocytes creep into the vascular endothelium or vessel walls. It has been also hypothesized that the macrophages creeping into the vascular endothelium and vessel walls become foam cells by uptaking oxidized LDL through the macrophage scavenger receptor, described above.

Formation of foam cells from macrophages at vessel walls is a primary cause of arteriosclerosis. Therefore, the assembling of monocytes and macrophages to the vascular endothelium, described above, is considered to trigger arteriosclerosis.

The oxidized-LDL receptor closely involved in the assembling of monocytes and macrophages to the vascular endothelium was first identified by the present inventors in 1997 after a long search by many researchers, and is currently in the limelight. Detailed studies on the oxidized-LDL receptor except for its characteristics and functions described above, are being vigorously done but there are hardly any reports yet. Therefore, the oxidized-LDL receptor is a novel technical field.

For conducting research and development on the oxidized-LDL receptor which is novel and clinically important for preventing and treating diseases such as arteriosclerosis, an assay system for investigating the interaction between various ligands including oxidized LDL in body fluids such as blood and the oxidized-LDL receptor is required, but it has not been provided yet.

To construct an assay system for a transmembrane protein such as the oxidized-LDL receptor, for example, producing its extracellular domain polypeptide as a soluble protein is necessary. Thanks to the recent development of genetic engineering techniques, the extracellular domain polypeptide can be exclusively produced as a recombinant soluble protein, however, for the convenience and applicability in purifying the recombinant protein and assaying (detection, quantification) using the recombinant protein, it is desirable to produce the extracellular domain as a soluble fusion polypeptide with another protein (for example, a part of immunoglobulin, such as Fc of immunoglobulin) [Unexamined Published Japanese Patent Application (JP-A) No. Hei 5-247094, International Patent Application Published in Japan No. Hei 3-502283, JP-A No. Hei 6-160395, etc].

A fusion polypeptide as such is not only effective as a component of an assay system, but is also useful for separating and purifying a denatured LDL such as the oxidized LDL and can be an effective ingredient of pharmaceuticals by itself.

However, regarding the oxidized-LDL receptor, any fusion polypeptide comprising such various uses has not been provided yet.

<u>Disclosure of the Invention</u>

Mechanisms of arteriosclerosis have not been clarified yet, and effective pharmaceuticals for preventing or treating arteriosclerosis have not been provided yet, either. As described above, an oxidized-LDL receptor is responsible for uptaking oxidized LDL in blood which triggers assembling of monocytes and macrophages to the vascular endothelium, an early response *in vivo* in foam cell formation process of macrophages, which is a causative of arteriosclerosis etc. Therefore, investigating functions of the oxidized-LDL receptor and the interaction between the oxidized-LDL receptor and various ligands including the oxidized LDL in blood is very important for developing pharmaceuticals effective for preventing and treating diseases such as arteriosclerosis and hyperlipidemia, etc.

Specifically, the present invention first provides, for the first time in the world, a soluble fusion polypeptide (a soluble oxidized-LDL receptor-fusion polypeptide) essential for achieving such an objective. The soluble oxidized-LDL receptor-fusion polypeptide, is useful not only as a component of an assay (detection, quantification, separation, purification, and such) for ligands such as oxidized LDL in body fluids (for example, in blood) of mammals (such as a normal individual and a patient), essential for achieving the objective, but also as an effective ingredient of pharmaceuticals for preventing and treating diseases such as arteriosclerosis and hyperlipidemia.

More specifically, the present invention provides a clinically applicable method for conveniently and sensitively detecting and quantifying a denatured LDL, such as an oxidized LDL in an intact condition existing in body fluids of a patient suffering from arteriosclerosis, hyperlipidemia as well as a kit used for the method.

As a result of zealous investigations by the present inventors relating to the soluble oxidized-LDL receptor, for use in research and development on the oxidized-LDL receptor clinically important for preventing and treating diseases such as arteriosclerosis, an oxidized-LDL receptor was successfully obtained using genetic engineering as a soluble fusion protein by producing it as a fusion polypeptide in which an extracellular domain of the oxidized-LDL receptor is linked to a part of an immunoglobulin (for example, Fc region), completing the present invention.

An extracellular domain of the oxidized-LDL receptor can be exclusively produced as a soluble polypeptide by treating the fusion polypeptide with a protease such as factor Xa etc.

The soluble fusion polypeptide of the oxidized-LDL receptor is useful not only as a component in assaying ligands such as an oxidized LDL in body fluids (for example in blood) of mammals (for example a normal individual and a patient) and separating and purifying the ligand, but also as an effective ingredient of pharmaceuticals for preventing and treating diseases such as arteriosclerosis and hyperlipidemia.

Moreover, by producing the above soluble oxidized-LDL receptor as a fusion polypeptide with a part (for example, Fc) of a constant region in an immunoglobulin such as IgG, the fusion polypeptide can be readily purified by means of an affinity column chromatography using characteristics of protein A specifically binding to a fragment of the immunoglobulin.

Further, as various antibodies against Fc of various immunoglobulins have been provided, an immunoassay for the fusion polypeptide can be conveniently performed by using an antibody against the Fc.

Specifically, the present invention is the invention described in the following (1) to (31).

(1) a fusion polypeptide comprising an extracellular domain of a mammalian oxidized-LDL receptor and a portion of a heavy chain in a mammalian immunoglobulin (Ig).

(2) the fusion polypeptide of (1), wherein the mammalian immunoglobulin is a human immunoglobulin.

(3) the fusion polypeptide of (1) or (2), wherein the immunoglobulin is IgG.

(4) the fusion polypeptide of any one of (1) to (3), wherein a portion of a immunoglobulin heavy chain is a constant region or a portion of constant region of an immunoglobulin heavy chain.

(5) the fusion polypeptide of (4), wherein a portion of a constant region comprises a hinge region, C2 domain and C3 domain of IgG, IgA or IgD.

(6) the fusion polypeptide of (4), wherein a portion of a constant region comprises C2 domain, C3 domain, and C4 domain of IgM or IgE.

(7) the fusion polypeptide of (1), wherein a portion of a heavy chain in the mammalian immunoglobulin comprises a hinge region, C2 domain and C3 domain of human IgG.

(8) the fusion polypeptide of any one of (1) to (7), wherein the mammalian oxidized-LDL receptor is a human oxidized-LDL receptor.

(9) the fusion polypeptide of (8), wherein the human oxidized-LDL receptor is a polypeptide comprising an amino acid sequence identical or substantially identical to the amino acid sequence of SEQ ID NO: 1.

(10) the fusion polypeptide of any one of (1) to (7), wherein the mammalian oxidized-LDL receptor is a bovine oxidized-LDL receptor.

(11) the fusion polypeptide of (10), wherein the bovine oxidized-LDL receptor is a polypeptide comprising an amino acid sequence identical or substantially identical to the amino acid sequence of SEQ ID NO: 2.

(12) a fusion polypeptide comprising an amino acid sequence identical or substantially identical to the amino acid sequence of SEQ ID NO: 3.

(13) a DNA encoding the fusion polypeptide of any one of (1) to (12).

(14) a DNA comprising the nucleotide sequence of SEQ ID NO: 4 or 10.

(15) a vector comprising a DNA of (13) or (14).

(16) a transformant transfected by the vector of (15).

(17) a fusion polypeptide-immobilized insoluble carrier, wherein the fusion polypeptide of any one of (1) to (12) is immobilized on an insoluble carrier.

(18) the fusion polypeptide-immobilized insoluble carrier of (17), wherein the insoluble carrier is selected from the group consisting of plates, test tubes, beads, balls, filters, and membranes.

(19) the fusion polypeptide-immobilized insoluble carrier of (17), wherein the insoluble carrier is a filter or a membrane, or one used for affinity column chromatography.

(20) a kit used for detecting or quantifying an oxidized LDL, comprising the fusion polypeptide-immobilized insoluble carrier of (17) or (18), or the fusion polypeptide of any one of (1) to (12).

(21) a method for detecting or quantifying an oxidized LDL by an immunoassay using the fusion polypeptide-immobilized insoluble carrier of (17) or (18), or the fusion polypeptide of any one of (1) to (12).

(22) the method for detecting or quantifying oxidized LDL by immunoassay of (21), comprising at least the following steps of (a) and (b);

(a) reacting a sample with the fusion polypeptide-immobilized insoluble carrier of (17) or (18), and,

(b) reacting the complex formed by binding of an oxidized LDL in the sample to the fusion polypeptide-immobilized insoluble carrier, with an antibody labeled with a labeling agent capable of providing a detectable signal by itself or by reacting with another substance, said antibody having reactivity to an oxidized LDL or apolipoprotein B.

(23) the method for detecting or quantifying an oxidized LDL by an immunoassay of (21) comprising at least the following steps of (a) and (b);

(a) reacting a sample with an antibody labeled with a labeling agent capable of providing a detectable signal by itself or by reacting with another substance, said antibody having reactivity to an oxidized LDL or apolipoprotein B, and

(b) reacting the complex formed by the binding between the antibody and an oxidized LDL in the sample, with the fusion polypeptide-immobilized insoluble carrier of (17) or (18).

(24) a method for detecting or quantifying an oxidized LDL by an immunoassay comprising at least the following step of (a);

(a) reacting a mixture comprising the fusion polypeptide-immobilized insoluble carrier of (17) or (18), an antibody labeled with a labeling agent capable of providing a detectable signal in itself or by reacting with another substance, said antibody having reactivity to an oxidized LDL or apolipoprotein B, and a sample.

(25) the method for detecting or quantifying an oxidized LDL by an immunoassay of (21) comprising at least the following step of (a);

(a) reacting the fusion polypeptide-immobilized insoluble carrier of (17) or (18), with a sample and an oxidized LDL standard labeled with a labeling agent capable of providing a detectable signal by itself or by reacting with another substance, said antibody having reactivity to an oxidized-LDL or apolipoprotein B.

(26) a kit used for separating or purifying an oxidized LDL, comprising the fusion polypeptide-immobilized insoluble carrier of (17) or (19).
(27) a method for separating or purifying an oxidized LDL, by affinity chromatography using the fusion polypeptide-immobilized insoluble carrier of (17) or (19).
(28) a method for purifying the oxidized LDL of (27), wherein the affinity chromatography is an affinity column chromatography.
(29) a pharmaceutical composition comprising a fusion polypeptide comprising an extracellular domain of a human oxidized-LDL receptor comprising the amino acid sequence of SEQ ID NO: 1, and a portion of a human immunoglobulin heavy chain, and a pharmaceutically acceptable carrier.
(30) the pharmaceutical composition of (29), wherein the portion of a immunoglobulin heavy chain is a constant region or a portion of the constant region of an immunoglobulin heavy chain.
(31) the pharmaceutical composition of (29) or (30), used for preventing or treating arteriosclerosis or hyperlipidemia.

The present invention is described in detail below, clarifying the fusion polypeptide and DNA of the present invention, a common method used for preparing an antibody used in the present invention, and the definitions of the terms used in the present invention.
"A mammal" in the present invention means a human, bovine, goat, rabbit, mouse, rat, hamster, and guinea pig, and preferably a human, bovine, rat, mouse or hamster, and more preferably a human or bovine.
"An oxidized-LDL receptor" of the present invention means an oxidized-LDL receptor derived from a "mammal" described above, comprising the amino acid sequence encoded by the DNA having the nucleotide sequence of SEQ ID NO: 5 or 6, or by the DNA hybridized with one of the DNAs under stringent conditions, and binding to a denatured LDL such as an oxidized LDL, or functioning in the uptake of the denatured LDL into cells. Preferably, it is a human or bovine oxidized-LDL receptor, and specifically, a human-derived polypeptide comprising an amino acid sequence identical or substantially identical to the amino acid sequence of SEQ ID NO: 1 (a human oxidized-LDL receptor), or a bovine derived polypeptide comprising an amino acid sequence identical or substantially identical to the amino acid sequence of SEQ ID NO: 2 (a bovine oxidized-LDL receptor). More preferably, it is a human derived polypeptide comprising the amino acid sequence of SEQ ID NO: 1 (a human oxidized-LDL receptor), or a bovine derived polypeptide comprising the amino acid sequence of SEQ ID NO: 2 (a bovine oxidized-LDL receptor).
Particularly, these two oxidized-LDL receptors are those reported in Nature (Vol. 386; p. 73-77, 1997), Shishitsu Seikagaku Kenkyu (Lipid Biochemistry Study) (Vol. 39, p83-84, 1997), or JP-A No. Hei 9-98787, and referred to as Ox-LDL Receptor or LOX-1 as well as Oxidized-LDL Receptor.
Examples of "stringent conditions" are as follows. When a probe with 50 or more nucleotides is used and hybridization is performed in 0.9% NaCl, the standard of temperature where a 50% dissociation occurs (Tm) can be calculated using the following formula and the temperature for hybridization determined according to the following formula.

$$Tm = 82.3°C + 0.41 \times (G+C)\ \% - 500/n - 0.61 \times (\text{formamide})\ \%$$

(n means the number of nucleotides of the probe).

$$\text{Temperature} = Tm - 25°C.$$

In addition, when a probe with 100 or more nucleotides (G+C = 40 to 50%) is used, it should be considered that Tm varies as (1) and (2) mentioned below.

(1) Tm descends by about 1°C per 1% mismatch.
(2) Tm descends by 0.6 to 0.7°C per 1% formamide.

Accordingly, the temperature conditions for the combination of completely complementary strands can be set as follows.

(A) 65 to 75°C (formamide not added)
(B) 35 to 45°C (in the presence of 50% formamide)

The temperature conditions for the combination of incompletely complementary strands can be set as follows.

(A) 45 to 55°C (formamide not added)
(B) 35 to 42°C (in the presence of 30% formamide)

The temperature conditions when a probe with 23 or less nucleotides is used can be 37°C or can be calculated using the following formula.

Temperature = 2°C x (the number of A+T) + 4°C x (the number of C+G) -5°C.

Here, "comprising an amino acid sequence-substantially identical" means to include a polypeptide having an amino acid sequence where a plurality of amino acids, preferably 1 to 10 amino acids, particularly preferably 1 to 5 amino acids, in the amino acid sequences shown in SEQ ID NO: 1, 2, or 3, are substituted, deleted, and/or modified, and a polypeptide having an amino acid sequence where a plurality of amino acids, preferably 1 to 10 amino acids, particularly preferably 1 to 5 amino acids, are added to said amino acid sequence, as long as the polypeptide has substantially the same biological properties as the polypeptide having said amino acid sequence.
The term "extracellular domain" used herein means the extracellular domain of "oxidized LDL receptors" defined as above.
The "extracellular domain" is explained as follows. Namely, besides the oxidized LDL receptor (Ox-LDL Receptor or LOX-1) described above, almost all receptors and cell surface molecules belong to transmembrane proteins and connect with the membrane through the hydrophobic peptide region penetrating the lipid bilayer of the membrane once or several times and has structure composed of three main domains, that is, extracellular domain, transmembrane domain, and cytoplasmic domain. Such a transmembrane protein exists as a monomer, homodimer, heterodimer, or oligomer with another chain(s) having the same or different amino acid sequence.
The term "extracellular domain" used herein means a whole or portion of the partial structure (partial sequence) of the transmembrane protein as mentioned above, which exists outside the membrane. In other words, it corresponds to a whole or portion of the region excluding the region incorporated into the membrane (transmembrane domain) and the region existing in the cytoplasm following the transmembrane domain (cytoplasmic domain).
Specifically, the extracellular domain of bovine LOX-1 that is included in the oxidized LDL receptor of the present invention means a whole or portion of the region excluding the amino acid residues 1 to 56 of the amino acid sequence set forth in SEQ ID NO: 2. For example, the domain encompasses a whole or portion of the region from any of the amino acid residues 57 to 65 to amino acid residue 270 in the following region. If desired, one to five amino acids can be added to the N-terminus and/or C-terminus of the extracellular domain.
The extracellular domain of human LOX-1 means a whole or portion of the region excluding the amino acid residues 1 to 59 of the amino acid sequence set forth in SEQ ID NO: 1. For example, the domain encompasses a whole or portion of the region from any of the amino acid residues 60 to 69 to amino acid residue 273 in the following region. If desired, one to five amino acids can be added to the N- terminus and/or C-terminus of the extracellular domain.
"A portion of the heavy chain of mammalian immunoglobulin (Ig)" used herein means a portion of the heavy chain (H chain) of immunoglobulin derived from mammals as described above, preferably that of human-derived immunoglobulin. The immunoglobulin can be any immunoglobulin belonging to any class and any subclass. Specifically, examples of the immunoglobulin are IgG (IgG1, IgG2, IgG3, and IgG4), IgM, IgA (IgA1 and IgA2), IgD, and IgE. Preferably, the immunoglobulin is IgG (IgG1, IgG2, IgG3, or IgG4), or IgM. Examples of particularly preferable immunoglobulin of the present invention are those belonging to human-derived IgG (IgG1, IgG2, IgG3, or IgG4).
As schematically shown in Fig. 1, immunoglobulin has a Y-shaped structural unit in which four chains composed of two homologous light chains (L chains) and two homologous heavy chains (H chains) are connected through disulfide bonds (S-S bonds). The light chain is composed of the light chain variable region ($V_L$) and the light chain constant region ($C_L$). The heavy chain is composed of the heavy chain variable region ($V_H$) and the heavy chain constant region ($C_H$).
The heavy chain constant region is composed of some domains having the amino acid sequences inherent in each class (IgG, IgM, IgA, IgD, and IgE) and each subclass (IgG1, IgG2, IgG3, and IgG4, IgA1, and IgA2).
The heavy chain of IgG (IgG1, IgG2, IgG3, and IgG4) is composed of $V_H$, $C_H1$ domain, hinge region, $C_H2$

domain, and $C_H3$ domain in this order from N terminus.

Similarly, the heavy chain of IgG1 is composed of $V_H$, $C\gamma_1 1$ domain, hinge region, $C\gamma_1 2$ domain, and $C\gamma_1 3$ domain in this order from N terminus. The heavy chain of IgG2 is composed of $V_H$, $C\gamma_2 1$ domain, hinge region, $C\gamma_2 2$ domain, and $C\gamma_2 3$ domain in this order from N terminus. The heavy chain of IgG3 is composed of $V_H$, $C\gamma_3 1$ domain, hinge region, $C\gamma_3 2$ domain, and $C\gamma_3 3$ domain in this order from N terminus. The heavy chain of IgG4 is composed of $V_H$, $C\gamma_4 1$ domain, hinge region, $C\gamma_4 2$ domain, and $C\gamma_4 3$ domain in this order from N terminus.

The heavy chain of IgA is composed of $V_H$, C $\alpha$ 1 domain, hinge region, C $\alpha$ 2 domain, and C $\alpha$ 3 domain in this order from N terminus.

Similarly, the heavy chain of IgA1 is composed of $V_H$, C $\alpha_1 1$ domain, hinge region, C $\alpha_1 2$ domain, and C $\alpha_1 3$ domain in this order from N terminus. The heavy chain of IgA2 is composed of $V_H$, C $\alpha_2 1$ domain, hinge region, C $\alpha_2 2$ domain, and C $\alpha_2 3$ domain in this order from N terminus.

The heavy chain of IgD is composed of $V_H$, C $\delta$ 1 domain, hinge region, C $\delta$ 2 domain, and C $\delta$ 3 domain in this order from N terminus.

The heavy chain of IgM is composed of $V_H$, C $\mu$ 1 domain, C $\mu$ 2 domain, C $\mu$ 3 domain, and C $\mu$ 4 domain in this order from N terminus and has no hinge region as seen in IgG, IgA, and IgD.

The heavy chain of IgE is composed of $V_H$, C $\varepsilon$ 1 domain, C $\varepsilon$ 2 domain, C $\varepsilon$ 3 domain, and C $\varepsilon$ 4 domain in this order from N terminus and has no hinge region as seen in IgG, IgA, and IgD.

If, for example, IgG is treated with papain, it is cleaved at the slightly N terminal side beyond the disulfide bonds existing in the hinge region where the disulfide bonds connect the two heavy chains to generate two homologous Fab, in which a heavy chain fragment composed of $V_H$ and $C_H1$ is connected with one light chain through a disulfide bond, and one Fc, in which two homologous heavy chain fragments each of which is composed of the hinge region, $C_H2$ domain, and $C_H3$ domain are connected through disulfide bonds (See "Immunology Illustrated", original 2nd ed., Nankodo, pp.65-75 (1992); and "Focus of Newest Medical Science 'Recognition Mechanism of Immune System'", Nankodo, pp.4-7 (1991); and so on).

Namely, "a portion of immunoglobulin heavy chain" of the present invention means a portion of an immunoglobulin heavy chain having the structural characteristics as mentioned above, and preferably, is a heavy chain constant region or its portion, more preferably the constant region without C1 domain, or the Fc region. Specifically, examples thereof are the region composed of hinge region, C2 domain, and C3 domain from each of IgG, IgA, and IgD, and are the region composed of C2 domain, C3 domain, and C4 domain from each of 1gM and IgE. A particularly preferable example thereof is the Fc region of human-derived IgG1.

The "fusion polypeptide" of the present invention is that composed of "the extracellular domain of the oxidized LDL receptor derived from a mammal" as defined above and "a portion of mammalian immunoglobulin (Ig) heavy chain." Preferably, it is a fusion polypeptide composed of an extracellular domain of the oxidized LDL receptor and a constant region of human immunoglobulin heavy chain or its portion, and particularly preferably, it is a fusion polypeptide composed of an extracellular domain of the oxidized LDL receptor and a constant region of human IgG heavy chain or its part, more preferably that composed of an extracellular domain of the oxidized LDL receptor and the region (Fc) composed of a hinge region, $C_H2$ domain, and $C_H3$ domain of human IgG heavy chain.

IgG is preferably IgG1. As the oxidized-LDL receptor, a human or bovine oxidized-LDL receptor is preferable, and the human oxidized-LDL receptor comprising the amino acid sequence of SEQ ID NO: 1, or the bovine oxidized-LDL receptor comprising the amino acid sequence of SEQ ID NO: 2 is more preferable. As an embodiment of the fusion polypeptide of the present invention relating to the bovine oxidized-LDL receptor, for example, the fusion polypeptide comprising the amino acid sequence of SEQ ID NO: 3 can be given. As a DNA encoding the fusion polypeptide of SEQ ID NO: 3, SEQ ID NO: 4 or 10 can be given.

The fusion polypeptide or the extracellular domain polypeptide of the present invention can be produced not only by recombinant DNA technology as mentioned below but also by a method well known in the art such as the chemical synthesis method and the cell culture method, or a modified method thereof.

The DNA of the present invention is a DNA encoding the fusion polypeptide of the present invention described above, and includes any nucleotide sequence capable of encoding the fusion polypeptide of the present invention. The DNA encoding the fusion polypeptide composed of an extracellular domain of a human or bovine oxidized-LDL receptors and a constant region or a part of a constant region of human immunoglobulin heavy chain is preferable. The DNA encoding the fusion polypeptide composed of an extracellular domain of a human or bovine oxidized-LDL receptor and a part of a constant region [particularly a region composed of $C_H2$ domain and $C_H3$ domain (Fc)] of a human IgG (particularly IgG1) is more preferable. A suitable example of a human oxidized-LDL receptor described above is a polypeptide comprising the amino acid sequence of SEQ ID NO: 1 and an example of a bovine oxidized-LDL receptor is the polypeptide of SEQ ID NO:2.

An embodiment of the DNA encoding the fusion polypeptide of the present invention relating to a bovine oxidized-LDL receptor is, for example, the DNA encoding the fusion polypeptide comprising the amino acid sequence of SEQ ID NO: 3, and the DNA comprising the nucleotide sequence of SEQ ID NO: 4 or 10.

As a DNA encoding an extracellular domain of an oxidized-LDL receptor, a part of the fusion polypeptide of the present invention, both cDNA and genomic DNA can be used.

A DNA encoding a portion of a immunoglobulin heavy chain, a part of the fusion polypeptide of the present invention, can be cDNA or genomic DNA comprising introns between each exon (for example, a DNA encoding $C_H1$ domain, hinge region, C $_H2$ domain, $C_H3$ domain, $C_H4$ domain, etc).

The DNA of the present invention includes any DNA composed of any codon that encodes the same amino acid.

The DNA of the present invention can be a DNA obtained by any method. For example, the DNA includes complementary DNA (cDNA) prepared from mRNA, DNA prepared from genomic DNA, DNA prepared by chemical synthesis, DNA obtained by PCR amplification with RNA or DNA as a template, and DNA constructed by appropriately combining these methods.

The DNA encoding the oxidized-LDL receptor and the immunoglobulin heavy chain in the present invention can be prepared by following standard methods: applying a method for cloning cDNA from mRNA encoding the oxidized-LDL receptor and immunoglobulin heavy chain, a method for isolating genomic DNA and splicing it, a method for preparing DNA by PCR using the cDNA sequence or mRNA sequence as a template, a method for chemical synthesis, and so on.

The DNA encoding the fusion polypeptide of the present invention can be prepared by cleaving (digesting) each DNA encoding the oxidized-LDL receptor and immunoglobulin heavy chain prepared in such a manner with an appropriate restriction enzyme, and linking the obtained DNA fragments, in combination with linker DNA or Tag if necessary, using an appropriate DNA polymerase and such.

cDNA encoding the oxidized LDL or immunoglobulin heavy chain (hereinafter referred to as the desired protein) can be cloned from mRNA by, for example, the method described below.

First, the mRNA encoding the desired protein is prepared from tissues or cells expressing and producing the desired protein. mRNA can be prepared by isolating total RNA by a known method such as quanidine-thiocyanate method (Chirgwin et al., Biochemistry, Vol 18, p5294, 1979), hot phenol method, or AGPC method, and subjecting it to affinity chromatography using oligo-dT cellulose or poly-U Sepharose.

Then, with the mRNA obtained as a template, cDNA is synthesized, for example, by a well-known method using reverse transcriptase, such as the method of Okayama et al (Mol. Cell. Biol. Vol.2, p.161 (1982); ibid. Vol.3, p.280 (1983)) or the method of Hoffman et al. (Gene Vol.25, p.263 (1983)), and converted into double-stranded cDNA. A cDNA library is prepared by transforming *E. coli* with plasmid vectors, phage vectors, or cosmid vectors having this cDNA or by transfecting *E coli* after *in vitro* packaging.

The plasmid vectors used in this invention are not limited as long as they are replicated and maintained in hosts. Any phage vector that can be replicated in hosts can also be used. Examples of usually used cloning vectors are pUC19, λ gt10, λ gt11, and so on. When the vector is applied to immunological screening as mentioned below, an vector having a promoter that can express a gene encoding the desired protein in a host is preferably used.

cDNA can be inserted into a plasmid by, for example, the method of Maniatis et al. (Molecular Cloning, A Laboratory Manual, second edition, Cold Spring Harbor Laboratory, p.1.53, 1989). cDNA can be inserted into a phage vector by, for example, the method of Hyunh et al. (DNA cloning, a practical approach, Vol.1, p.49 (1985)). These methods can be simply performed by using a commercially available cloning kit (for example, a product from Takara Shuzo). The recombinant plasmid or phage vector thus obtained is introduced into an appropriate host cell such as a prokaryote (for example, *E. coli*: HB101, DH5 α, DH10B, MC1061/P3, etc).

Examples of a method for introducing a plasmid into a host are, calcium chloride method, calcium chloride/rubidium chloride method described in Molecular Cloning, A Laboratory Manual (second edition, Cold Spring Harbor Laboratory, p.1.74 (1989)), electroporation method, and so on. Phage vectors can be introduced into host cells by, for example, a method in which the phage DNAs are introduced into grown hosts after *in vitro* packaging. *In vitro* packaging can be easily performed with a commercially available *in vitro* packaging kit (for example, a product from Stratagene or Amersham).

The cDNA encoding the desired protein can be isolated from the cDNA library so prepared according to the method mentioned above by combining general cDNA screening methods.

For example, a clone comprising the desired cDNA can be screened by a known colony hybridization method (Crunstein et al. Proc. Natl. Acad. Sci. USA, Vol.72, p.3961 (1975)) or plaque hybridization method (Molecular Cloning, A Laboratory Manual, second edition, Cold Spring Harbor Laboratory, p.2.108 (1989)) using $^{32}$P-labeled chemically synthesized oligonucleotides as probes, which correspond to the amino acid sequence of the desired protein. Alternatively, such a clone can be obtained by isolating a clone comprising a DNA fragment encoding a specific region within the desired protein by amplifying the region by PCR with synthetic PCR primers.

When a cDNA library prepared using a cDNA expression vector (for example, λ ZAPII phage vector) is used, the desired clone can be screened by the antigen-antibody reaction using an antibody against the desired protein. A screening method using PCR method is preferably used when many clones are subjected to screening.

The nucleotide sequence of the DNA thus obtained can be determined by Maxam-Gilbert method (Maxam et al. Proc. Natl. Acad. Sci. USA, Vol.74, p.560 (1977)) or the dideoxynucleotide synthetic chain termination method using phage M13 (Sanger et al. Proc. Natl. Acad. Sci. USA, Vol.74, pp.5463-5467 (1977)). The gene encoding the desired protein can be obtained by excising the clone to obtain a whole or portion of the clone as mentioned above with restriction enzymes and so on.

Also, the DNA encoding the desired protein can be isolated from the genomic DNA derived from the cells expressing the desired protein as mentioned above by the following methods.

Such cells are solubilized preferably by SDS or proteinase K, and the DNAs are deproteinized by repeating phenol extraction. DNAs are digested preferably with ribonuclease. The DNAs obtained are partially digested with appropriate restriction enzymes, and the DNA fragments obtained are amplified with appropriate phage or cosmid to generate a library. Then, clones having the desired sequence are detected, for example, by using radioactively labeled DNA probes, and a whole or part of the gene encoding the desired protein is obtained from the clones by excision with restriction enzyme and so on.

cDNA encoding a human-derived protein can be obtained by preparing a cosmid library into which human genomic DNAs (chromosomal DNAs) are introduced ("Laboratory Manual Human Genome Mapping," M. Hori and Y. Nakamura, eds., Maruzen), screening the cosmid library to obtain positive clones containing DNA corresponding to the coding region of the desired protein, and screening the above cDNA library using the coding region DNA excised from the positive clones as a probe.

A DNA encoding a desired protein can be prepared by following standard PCR methods using known mRNA or cDNA of the desired protein as a template (Gene Amplification PCR method, Basics and Novel Development, Kyoritsu Publishers, 1992, etc).

A DNA encoding the desired protein can be chemically synthesized based on the nucleotide sequence of the desired protein by following the standard methods.

The fusion polypeptide of the present invention can be prepared as a recombinant protein using common gene engineering technologies by following standard methods: obtaining the DNA fragment encoding the extracellular domain of the oxidized-LDL receptor and a portion of a immunoglobulin heavy chain by digesting the DNA (cDNA or genomic DNA including introns) encoding the oxidized-LDL receptor and the immunoglobulin heavy chain prepared by the methods illustrated above, with each appropriate restriction enzyme, and linking the fragments by adding an appropriate restriction enzyme site at the ends, or using linker DNA or Tag if necessary, with an appropriate DNA polymerase and such, to prepare the fused DNA.

Specifically, the following are the examples.

The DNA (preferably cDNA) encoding an extracellular domain of an oxidized-LDL receptor can be prepared by digesting a nucleotide sequence around the boundary between the extracellular and transmembrane domains of a target oxidized-LDL receptor with an appropriate restriction enzyme capable of digesting at an appropriate site. For example, in the case of the cDNAs encoding the human and bovine oxidized-LDL receptors LOX-1 (SEQ ID NOs: 1 and 2), *Bam*HI can be used.

The DNA encoding a portion of a immunoglobulin heavy chain can be prepared by digesting with a restriction enzyme capable of digesting at a desired site. For example, the cDNA encoding Fc region of the human IgG1 can be prepared by using *Bam*HI.

Linkage between the DNA encoding an extracellular domain of an oxidized-LDL and the DNA encoding a portion of a immunoglobulin heavy chain obtained in the above manner can be done by adding an appropriate restriction enzyme site at the ends, or applying a commercialized DNA ligation kit, in combination with linker DNA or Tag if necessary.

A transformant can be prepared by constructing the expression vector by inserting the fused DNA prepared in such a manner to a vector such as those described below, and transfecting the host cells described below with the expression vector. The fusion polypeptide can be produced in culture supernatants by culturing the transformants. The fusion polypeptide in the culture supernatant can be readily purified using the protein A column chromatography and so on.

The present invention also relates to an expression vector comprising the DNA encoding the fusion polypeptide of the present invention. As an expression vector of the present invention, any vector can. be used as long as it is capable of retaining replication or self-multiplication in each host cell of prokaryotic and/or eukaryotic cells, including plasmid vectors and phage vectors (Cloning Vectors: A laboratory Manual, Elsevier, New York, 1985).

The recombinant vector can easily be prepared by ligating the DNA encoding the fusion polypeptide of the present invention with a vector for recombination available in the art (plasmid DNA and bacteriophage DNA) by the usual method. Specific examples of the vectors for recombination used are *E. coli*-derived plasmids such as pBR322, pBR325, pUC12, pUC13, and pUC 19, yeast-derived plasmids such as pSH19 and pSH15, and *Bacillus subtilis*-derived plasmids such as pUB110, pTP5, and pC194. Examples of phages are a bacteriophages such as λ phage, and an animal or insect virus (pVL1393, Invitrogen) such as a retrovirus, vaccinia virus, and nuclear polyhedrosis virus.

A plasmid vector is useful for expressing the DNA encoding the fusion polypeptide of the present invention and for producing the fusion polypeptide. The plasmid vector is not limited as long as it expresses the gene encoding the fusion polypeptide in various prokaryotic and/or eukaryotic host cells and produces this polypeptide. Examples thereof are pMAL C2, pEF-BOS (Nucleic Acids Res. Vol.18, p.5322 (1990)), pME18S (Experimental Medicine: SUPPLEMENT, "Handbook of Genetic Engineering" (1992)), and so on.

When bacteria, particularly *E. coli* are used as host cells, an expression vector is generally comprised of, at least, a promoter/operator region, an initiation codon, the DNA encoding the protein of the present invention, termination codon, terminator region, and replicon.

When yeast, animal cells, or insect cells are used as hosts, an expression vector is preferably comprised of, at least, a promoter, an initiation codon, the DNA encoding the fusion polypeptide of the present invention, and a termination codon. It may also comprise the DNA encoding a signal peptide, enhancer sequence, 5'- and 3'-untranslated region of the gene encoding the fusion polypeptide of the present invention, splicing junctions, polyadenylation site, selectable marker region, and replicon. The expression vector may also contain, if required, a gene for gene amplification (marker) that is usually used.

A promoter/operator region to express the fusion polypeptide of the present invention in bacteria comprises a promoter, an operator, and a Shine-Dalgarno (SD) sequence (for example, AAGG). For example, when the host is *Escherichia*, it preferably comprises Trp promoter, lac promoter, recA promoter, λ PL promoter, lpp promoter, tac promoter, or the like.

Examples of a promoter to express the fusion polypeptide of the present invention in yeast are PH05 promoter, PGK promoter, GAP promoter, ADH promoter, and so on. When the host is Bacillus, examples thereof are SL01 promoter, SP02 promoter, penP promoter and so on.

When the host is a eukaryotic cell such as a mammalian cell, examples thereof are SV40-derived promoter, retrovirus promoter, heat shock promoter, and so on, and preferably SV-40 and retrovirus-derived one. As a matter of course, the promoter is not limited to the above examples. In addition, using an enhancer is effective for expression.

A preferable initiation codon is, for example, a methionine codon (ATG).

The commonly used termination codon (for example, TAG, TGA, TAA, and so on) is illustrated as a termination codon.

Usually used natural or synthetic terminators are used as a terminator region.

A replicon means a DNA capable of replicating the whole DNA sequence in host cells, and includes a wild-type plasmid, an artificially modified plasmid (DNA fragment prepared from a wild-type plasmid), a synthetic plasmid, and so on. Examples of preferable plasmids are pBR322 or its artificial derivatives (DNA fragment obtained by treating pBR322 with appropriate restriction enzymes) for *E. coli*, yeast 2 μ plasmid or yeast chromosomal DNA for yeast, and pRSVneo (ATCC 37198), pSV2dhfr (ATCC 37145), pdBPV-MMTneo (ATCC 37224), pSV2neo (ATCC 37149), pSV2bsr, and such for mammalian cells.

An enhancer sequence, polyadenylation site, and splicing junction that are usually used in the art, such as those derived from SV40 can be also used.

A selectable marker usually employed can be used according to the usual method. Examples thereof are resistance genes for antibiotics, such as tetracycline, neomycin, ampicillin, or kanamycin, and thymidine kinase gene.

Examples genes for gene amplification are dihydrofolate reductase (DHFR) gene, thymidine kinase gene, neomycin resistance gene, glutamate synthase gene, adenosine deaminase gene, ornithine decarboxylase gene, hygromycin-B-phophotransferase gene, aspartate transcarbamylase gene, and such.

The expression vector of the present invention can be prepared by continuously and circularly linking at least the above-mentioned promoter, initiation codon, DNA (gene) encoding the polypeptide of the present invention, termination codon, and terminator region, to an appropriate replicon. If desired, appropriate DNA fragments (for example, linkers, restriction sites generated with other restriction enzyme), can be used by the usual method such as digestion with a restriction enzyme or ligation using T4 DNA ligase.

Transformants of the present invention can be prepared by introducing the expression vector mentioned above into host cells.

Host cells used in the present invention are not limited as long as they are compatible with an expression vector mentioned above and can be transformed. Examples thereof are various cells such as wild-type cells or artificially established recombinant cells usually used in technical field of the present invention (for example, bacteria (*Escherichia and Bacillus*), yeast (*Saccharomyces*, *Pichia*, and such), animal cells, or insect cells.

*E. coli* or animal cells are preferably used. Specific examples are *E. coli* (DH5 α, DH10B, TB1, HB101, XL-2Blue, and such); mouse-derived cells (COP, L, C127, Sp2/0, NS-1, NIH 3T3, and such), rat-derived cells, hamster-derived cells (BHK, CHO, and such), monkey-derived cells (COS1, COS3, COS7, CV1, Velo, and such), and human-derived cells (Hela, diploid fibroblast-derived cells, myeloma, Namalwa, and such).

An expression vector can be introduced (transformed (transduced)) into host cells by known methods.

Transformation can be performed, for example, according to the method of Cohen et al. (*Proc. Natl. Acad.*

*Sci.* USA, Vol.69, p.2110 (1972)), protoplast method (Mol. Gen. Genet., Vol.168, p.111 (1979)), or competent method (J. Mol. Biol., Vol.56, p.209 (1971)) when the hosts are bacteria (*E. coli, Bacillus subtilis*, and such), the method of Hinnen et al. (Proc. Natl. Acad. Sci. USA, Vol.75, p.1927 (1978)), or lithium method (J. Bacteriol., Vol.153, p.163 (1983)) when the host is *Saccharomyces cerevisiae*, the method of Graham (Virology, Vol.52, p.456 (1973)) when the hosts are animal cells, and the method of Summers et al. (Mol. Cell. Biol., Vol.3, pp.2156-2165 (1983)) when the hosts are insect cells.

The fusion polypeptide of the present invention can be produced by cultivating transformants (in the following this term includes transductants) comprising an expression vector prepared as mentioned above in nutrient media.

The nutrient media preferably comprise carbon source, inorganic nitrogen source, or organic nitrogen source necessary for the growth of host cells (transformants). Examples of the carbon source are glucose, dextran, soluble starch, and sucrose, and examples of the inorganic or organic nitrogen source are ammonium salts, nitrates, amino acids, corn steep liquor, peptone, casein, meet extract, soy bean cake, and potato extract, If desired, they may comprise other nutrients (for example, an inorganic salt (for example, calcium chloride, sodium dihydrogenphosphate, and magnesium chloride), vitamins, antibiotics (for example, tetracycline, neomycin, ampicillin, kanamycin, and so on).

Cultivation is performed by a method known in the art. Cultivation conditions such as temperature, pH of the media, and cultivation time are selected appropriately so that the protein of the present invention is produced in large quantifies.

Specific media and cultivation conditions used depending on host cells are illustrated below, but are not limited thereto.

When the hosts are bacteria, actinomycetes, yeasts, filamentous fungi, liquid media comprising the nutrient source mentioned above are appropriate. The media with pH 5 to 8 are preferably used.

When the host is *E. coli*, examples of preferable media are LB media, M9 media (Miller et al. Exp. Mol. Genet., Cold Spring Harbor Laboratory, p.431 (1972)) and Y.T. media. Using these media, cultivation can be performed usually at 14 to 43 °C for about 3 to 24 hours with aeration and stirring, if necessary.

When the host is *Bacillus*, cultivation can be performed usually at 30 to 40 °C for about 16 to 96 hours with aeration and stirring, if necessary.

When the host is yeast, an example of media is Burkholder minimal media (Bostian, Proc. Natl. Acad. Sci. USA, Vol.77, p.4505 (1980)). The pH of the media is preferably 5 to 8. Cultivation can be performed usually at 20 to 35°C for about 14 to 144 hours with aeration and stirring, if necessary.

When the host is an animal cell, examples of media are MEM media containing about 5 to 20% fetal bovine serum (Science, Vol.122, p.501 (1952)), DMEM media (Virology, Vol.8, p.396 (1959)), RPMI1640 media (J. Am. Med. Assoc., Vol.199, p.519 (1967)), 199 media (Proc. Soc. Exp. Biol. Med., Vol.73, p.1 (1950)), HamF12 media, and so on. The pH of the media is preferably about 6 to 8. Cultivation can be performed usually at about 30 to 40°C for about 15 to 72 hours with aeration and stirring, if necessary.

When the host is an insect cell, an example of media is Grace's media containing fetal bovine serum (Proc. Natl. Acad. Sci. USA, Vol.82, p.8404 (1985)). The pH thereof is preferably about 5 to 8. Cultivation can be performed usually at about 20 to 40°C for 15 to 100 hours with aeration and stirring, if necessary.

The fusion polypeptide of the present invention can be produced by cultivating transformants as mentioned above, in particular animal cells or *E. coli*, to secrete the polypeptide into the culture supernatant. Namely, a culture filtrate (supernatant) is obtained by a method such as filtration or centrifugation of the obtained culture, and the fusion polypeptide of the present invention is purified and isolated from the culture filtrate by methods commonly used in order to purify and isolate a natural or synthetic protein.

Examples of the isolation and purification method are a method utilizing affinity, such as protein A affinity chromatography; a method utilizing solubility, such as salting out and solvent precipitation method; a method utilizing, the difference in molecular weight, such as dialysis, ultrafiltration, gel filtration, and sodium dodecyl sulfate-polyacrylamide gel electrophoresis; a method utilizing charges, such as ion exchange chromatography and hydroxylapatite chromatography; a method utilizing the difference in hydrophobicity, such as reverse phase high performance liquid chromatography; and a method utilizing the difference in isoelectric point, such as isoelectric focusing.

When the fusion polypeptide of the present invention exists in the periplasm or cytoplasm of cultured transformants, first, the fungus bodies or cells are harvested by the usual method such as filtration or centrifugation and suspended in appropriate buffer. After the cell wall and/or cell membrane of the cells and so on are disrupted by the method such as lysis with sonication, lysozyme, and freeze-thawing, the membrane fraction comprising the fusion polypeptide of the present invention is obtained by the method such as centrifugation or filtration.

The membrane fraction is solubilized with a detergent such as Triton-X100 to obtain the crude extract. Finally, the polypeptide is isolated and purified from the crude extract by the usual method as illustrated above.

An "antibody" used herein means a polyclonal (antiserum) or a monoclonal antibody. The present invention also comprises a portion of the monoclonal antibody such as Fab as described below.

Specifically, the antibody of the present invention is an antibody with a reactivity to various denatured LDLs (oxidized LDL, acetyl LDL, succinyl LDL, malonedialdehyde LDL, and so on), such as an oxidized LDL of a mammal (a human, bovine, mouse, rat, hamster, guinea pig, and a rabbit, and preferably a human), or apolipoprotein B and preferably it is an antibody with a reactivity to at least apolipoprotein B of the mammals (preferably humans).

Specifically, as an LDL (low density lipoprotein) is bound to apolipoprotein B in body fluids such as blood, an antibody with a reactivity to various denatured LDLs such as an oxidized LDL or an antibody with the reactivity to apolipoprotein B can be used in the present invention,

As the antibody, any antibody can be used as long as it has a reactivity to various denatured LDLs such as the oxidized LDL, or to apolipoprotein B (including recombinant proteins). Specifically, any natural mammalian antibody obtained by immunizing a mammal such as a mouse, a rat, hamster, guinea pig, rabbit, goat, sheep and such with various denatured LDLs such as the oxidized LDL or apolipoprotein B (including recombinant proteins); a chimera antibody obtained by using gene engineering techniques (Experimental Medicine, supplement, Vol. 1.6, No. 10, 1988, Examined Published Japanese Patent Application (JP-B) No. Hei 3-73280, Molecular Medicine, Vol. 32, No. 6, p638-644, 1995 and so on); and a humanized antibody ((CDR-grafted antibody, International Patent Application Published in Japan No. Hei 4-506458, JP-A No. Sho 62-296890, Molecular Medicine, Vol. 32, No. 6, p638-644, 1995, and so on) can be used.

The monoclonal antibody includes those belonging to any class or subclass of IgG (IgG1, IgG2, IgG3, IgG4), IgM, IgA (IgA1, IgA2), IgD, or IgE. IgG or IgM is preferable.

The polyclonal antibody (antiserum) or monoclonal antibody of the present invention can be produced by the known methods. Namely, a mammal, preferably, a mouse, rat, hamster, guinea pig, rabbit, cat, dog, pig, goat, sheep, horse, or bovine, or more preferably, a mouse, rat, hamster, guinea pig, rabbit, goat or sheep is immunized, for example, with an antigen mentioned above with Freund's adjuvant, if necessary.

The polyclonal antibody can be obtained from the antiserum obtained from the animal so immunized. In addition, the monoclonal antibodies are produced as follows. Hybridomas are prepared by cell fusion between the antibody-producing cells obtained from the animal so immunized and myeloma cells that are not capable of producing autoantibodies. The hybridomas are cloned, and clones producing the monoclonal antibodies showing the specific affinity to the antigen used for immunizing the mammal are screened.

Specifically, the monoclonal antibody can be produced as follows. Immunizations are performed by injecting or implanting once or several times a modified LDL as described above including oxidized LDL or apolipoprotein B (including recombinant protein) as an immunogen, if necessary, with Freund's adjuvant, subcutaneously, intramuscularly, intravenously, through the footpad, or intraperitoneally into a mouse, rat, hamster, guinea pig, rabbit, goat or sheep. Usually, immunizations are performed once to four times every one to fourteen days after the first immunization. Antibody-producing cells are obtained from the mammal so immunized, if necessary, in about one to five days after the last immunization.

Hybridomas that secrete a monoclonal antibody can be prepared by the method of Köhler and Milstein (Nature, Vol.256, pp.495-497 (1975)) and by its modified method. Namely, hybridomas are prepared by fusing antibody-producing cells contained in a spleen, lymph node, bone marrow, or tonsil obtained from the mammal immunized as mentioned above, preferably a spleen, with myelomas without autoantibody-producing ability, which are derived from, preferably, a mammal such as a mouse, rat, guinea pig, hamster, rabbit, or human, or more preferably, a mouse, rat, or human.

For example, mouse-derived myeloma P3/X63-AG8.653 (653), P3/NSI/1-Ag4-1 (NS-1), P3/X63-Ag8.U1 (P3U1), SP2/0-Ag14 (Sp2/0, Sp2), PAI, F0, or BW5147, rat-derived myeloma 210RCY3-Ag.2.3., or human-derived myeloma U266AR1, GM1500-6TG-A1-2, UC729-6, CEM-AGR, D1R11, or CEM-T15 can be used as a myeloma used for the cell fusion.

Hybridoma clones producing monoclonal antibodies can be screened by cultivating hybridomas, for example, in microtiter plates and by measuring the reactivity of the culture supernatant in the well in which hybridoma growth is observed, to the immunogen used for the immunization mentioned above, for example, by enzyme immunoassay such as RIA and ELISA.

The monoclonal antibodies can be produced from hybridomas by cultivating the hybridomas *in vitro* or *in vivo* such as in the ascites fluid of a mouse, rat, guinea pig, hamster, or rabbit, preferably a mouse or rat, more preferably mouse and isolating the antibodies from the resulting the culture supernatant or ascites fluid of a mammal.

Cultivating hybridomas *in vitro* can be performed depending on the property of cells to be cultured, on the object of a test study, and on the various conditions of a cultivating method, by using known nutrient media or any nutrient media derived from known basal media for growing, maintaining, and storing the hybridomas to produce monoclonal antibodies in culture supernatant.

Examples of basal media are low calcium concentration media such as Ham'F12 medium, MCDB153 medium, or low- calcium concentration MEM medium, and high calcium concentration media such as MCDB104 medium, MEM medium, D-MEM medium, RPMI1640 medium, ASF104 medium, or RD medium. The basal media can contain, for example, sera, hormones, cytokines, and/or various inorganic or organic substances depending on the objective.

Monoclonal antibodies can be isolated and purified from the culture supernatant or ascites fluid mentioned above by saturated ammonium sulfate precipitation, euglobulin precipitation method, caproic acid method, caprylic acid method, ion exchange chromatography (DEAE or DE52), affinity chromatography using anti-immunoglobulin column or protein A column.

The "portion of a monoclonal antibody" used in the present invention means $F(ab')_2$, Fab', Fab, Fv (variable fragment of antibody), sFv, dsFv (disulfide stabilized Fv), dAb (single domain antibody), and such (Exp. Opin. Ther. Patents, Vol.6, No.5, pp.441-456 (1996)).

" $F(ab')_2$" and " Fab' " can be produced by treating immunoglobulin (monoclonal antibody) with a protease such as pepsin and papain, and means an antibody fragment generated by digesting immunoglobulin near the disulfide bonds existing between the hinge regions in each of the two H chains. For example, papain cleaves IgG upstream of the disulfide bonds existing between the hinge regions in each of the two H chains to generate two homologous antibody fragments in which an L chain composed of $V_L$ (L chain variable region) and $C_L$ (L chain constant region), and an H chain fragment composed of $V_H$ (H chain variable region) and $C_H\gamma 1$ ($\gamma 1$ region in the constant region of H chain) are connected at their C terminal regions through a disulfide bond. Each of such two homologous antibody fragments is called Fab'. Pepsin also cleaves IgG downstream of the disulfide bonds existing between the hinge regions in each of the two H chains to generate an antibody fragment slightly larger than the fragment in which the two above-mentioned Fab' are connected at the hinge region. This antibody fragment is called $F(ab')_2$.

The term "insoluble carrier" as referred to in the present invention indicates a supporting material thereon used for immobilizing the fusion polypeptide of the present invention by physical adsorption or chemical bonding.

The insoluble carrier is exemplified below in (A) and (B):

(A) plastics such as polystyrene resin, polycarbonate resin, silicone resin or nylon resin; plates made of water-insoluble material represented by glass; containers having internal spaces such as test tubes or tubes; beads; balls; filters or membranes;

(B) insoluble carriers, used for affinity chromatography, such as cellulose carriers, agarose carriers, polyacrylamide carriers, dextran carriers, polystyrene carriers, polyvinyl alcohol carriers, poly(amino acid) carriers or porous silica carriers.

The term" fusion polypeptide-immobilized insoluble carrier" as referred to in the present invention indicates the above-defined insoluble carrier on which the fusion polypeptide of this invention is immobilized by physical adsorption or chemical bonding. These fusion polypeptide-immobilized insoluble carriers can be used for the detection, quantification, separation or purification of denatured LDL such as oxidized LDL in samples (for example, body fluids such as serum and plasma, culture supernatants, the supernatant fluids obtained by centrifugation and so on).

The insoluble carriers shown above in (A) can be used for the detection and the quantification; from the standpoint of the simplicity of operation and the simultaneous processing of many samples, in particular, the multi-well microtiter plates, which are made of plastics and have many wells, such as 96-well microtiter plates or 48-well microtiter plates, are used preferably as an insoluble carrier in the assay for quantification.

The filters or membranes shown above in (A), or the insoluble carriers shown above in (B), can be used for the separation or the purification.

A "labeling agent capable of providing a detectable signal by itself or by reacting with another substance" as referred to in this invention means a substance used for converting the antibodies described above, or a standard of oxidized LDL into detectable forms by binding thereto by physical or chemical bonding. Specifically, the labeling substance includes enzymes, fluorescent materials, chemiluminescent materials, biotin, avidin or radioisotopes, and so on, more specifically, enzymes such as peroxidase (for example, horseradish peroxidase), alkaline phosphatase, $\beta$ -D-galactosidase, glucose oxidase, glucose-6-phosphate dehydrogenase, alcohol dehydrogenase, malate dehydrogenase, penicillinase, catalase, apo-glucose oxidase, urease, luciferase or acetylcholinesterase; fluorescent materials such as fluorescein isothiocyanate, phycobiliprotein, chelating compounds of the rare-earth metals, dansyl chloride or tetramethylrhodamine isothiocyanate; radioisotopes such as $^{3}H$, $^{14}C$, $^{125}I$ or $^{131}I$; biotin; avidin; or chemiluminescent materials.

Radioisotopes and fluorescent materials, even when used alone, give a detectable signal. On the other hand, enzymes, chemiluminescent materials, biotin, and avidin give no detectable signal, when used alone. In these cases, one or more substances are reacted with the substances to give a detectable signal. For example, when the substance is an enzyme, at least a substrate for the enzyme is necessary to give a detectable signal. Various types of substrates are selectable depending on the methods for measuring the enzyme activity (colorimetry, immunofluorescence method, bioluminescence method, chemiluminescence method, and so on). For example, hydrogen peroxide is used as a substrate for peroxidase. When biotin is selected, avidin or enzyme-conjugated avidin is used for the reaction with biotin generally but not always. According to the need, various coloring agents are further used for the reaction depending on the type of the substrate.

Any labeling agent can be used in the present invention but when considering the sensitivity of detection and quantification and convenience of handling, an enzyme such as peroxidase or biotin is preferable.

The term, "a standard of oxidized LDL (oxidized LDL standard)" as referred to in the present invention indicates a denatured LDL such as oxidized LDL isolated previously, which differs from a denatured LDL such as oxidized LDL with an unknown concentration (content) in a sample, and a standard which can be adjusted to any desired concentration according to the purpose of the assay. For example, the standard substance can be used for the preparation of calibration curves.

An "immunoassay" of the present invention means a method for detecting or quantifying a target substance in a sample (for example, a body fluid sample such as serum, plasma, culture supernatant or centrifuge supernatant, and so on) based on the principles of the receptor-ligand reactions and antibody-antigen reactions. In the current invention, the receptor is an oxidized-LDL receptor and the ligand is a ligand against the oxidized-LDL (a denatured LDL such as an oxidized LDL) receptor. In the present invention, the antigen is a ligand (a denatured LDL such as an oxidized LDL) against an oxidized-LDL receptor, and the antibody is an antibody with a reactivity to the ligand (a denatured LDL such as an oxidized-LDL) against the oxidized-LDL receptor or an antibody with a reactivity to apolipoprotein B, which is capable of binding to a denatured LDL such as the oxidized-LDL. In this invention, any known immunoassay can be used as long as it is a method that can implement receptor-ligand reaction, and the antigen-antibody reaction.

Specifically, principles of various methods such as those described in Enzyme immunoassays (the third edition, edited by E. Ishikawa et al, Igakushoin, 1987) can be applied. In the various methods, for capturing or trapping a target substance to be detected or quantified in a sample, more than one antibody against the target substance is used. In the present invention, an assay can be carried out by replacing one of the antibodies by a fusion polypeptide of the present invention.

As applicable principles, for example, single antibody solid-phase method, two antibodies liquid-phase method, two antibodies solid-phase method, sandwich method, and one pot method such as described in JP-B No. Hei 2-39747 are suitable examples. As an assay utilizing the antigen-antibody reaction, enzyme multiplied immunoassay technique (EMIT technique), enzyme channeling immunoassay, enzyme modulator mediated enzyme immunoassay (EMMIA), enzyme inhibitor immunoassay, immunoenzymetric assay, enzyme enhanced immunoassay, proximal linkage immunoassay, and so on are also known.

In the present invention, any one of the principles of these immunoassays can be selected and used depending on the purpose, however, when considering operational convenience and/or economical convenience, and especially clinical applicability, use of principles of the sandwich method, one pot method or single antibody solid-phase method is preferable, and sandwich method or one pot method is more preferable. The sandwich method using the fusion polypeptide-immobilized insoluble carrier wherein the polypeptide of the present invention is fixed on a multi-well microtiter plate, represented by a 96-well microtiter plate, and an antibody labeled with enzyme or biotin, or the one pot method using beads, on the surface of which the fusion polypeptide of the present invention is immobilized and an antibody labeled with an enzyme such as peroxidase or biotin are particularly preferable.

One example of suitable embodiments of the present invention is the sandwich method or one pot method using the fusion polypeptide-immobilized insoluble carrier on which the fusion polypeptide composed of an extracellular domain of a human or a bovine oxidized-LDL receptor (preferably LOX-1), and a portion of a constant region (preferably Fc) of the heavy chain of a human immunoglobulin (preferably IgG, and more preferably IgG1) is fixed on a multi-well microtiter plate or beads, and the antibody that has a reactivity to a denatured LDL such as an oxidized LDL or apolipoprotein B and is labeled with an enzyme or biotin.

Methods applying the principles of the sandwich method, the one pot method and the single antibody solid-phase method are illustrated below.

A method applying the principle of the sandwich method is the method of (22) described above, specifically, the immunoassay method comprising at least the following processes of (a) and (b):

(a) reacting a sample with the fusion polypeptide-immobilized insoluble carrier of the present invention, and
(b) reacting the complex formed by binding of the oxidized LDL in the sample to the fusion polypeptide-immobilized insoluble carrier, with an antibody labeled with a labeling agent capable of providing a detectable signal by itself or by reacting with another substance, said antibody having a reactivity to an oxidized LDL or apolipoprotein B.

A specific example of a assaying method of the present invention in which the "insoluble carrier" is a multi-well microtiter plate and the "labeling agent" is an enzyme such as peroxidase or biotin, comprises, for example, the steps as described below, but the method is not to be construed as being restricted to the specific example.

(Step 1) preparing a fusion polypeptide-immobilized multi-well microtiter plate by immobilizing the fusion polypeptide of the present invention on a multi-well microplate;
(Step 2) reacting a sample such as human plasma with the fusion polypeptide immobilized on the microplate by adding the sample to the microplate;

(Step 3) washing out the unreacted sample from the microplate;

(Step 4) preparing a labeled antibody by labeling an antibody having a reactivity to oxidized LDL or apolipoprotein B with biotin or an enzyme such as peroxidase;

(Step 5) reacting the labeled antibody with the complex formed through the reaction between a denatured LDL such as oxidized LDL in the sample with the fusion polypeptide immobilized on the microplate, by adding the labeled antibody to the microplate washed in Step 3;

(Step 6) washing out the unreacted labeled antibody from the microplate;

(Step 7) reacting the labeling agent moiety of the labeled antibody with a substrate selected depending on the type of the enzyme used (when the labeled antibody used in Step 5 is labeled with an enzyme such as peroxidase), avidin or enzyme-conjugated avidin (when the labeled antibody used in Step 5 is labeled with biotin), by adding, if necessary together with a coloring agent, the substrate, or avidin or enzyme-conjugated avidin to the microplate washed in Step 6;

(Step 8) reacting the enzyme conjugated with avidin with a substrate for the enzyme selected depending on the type of the enzyme conjugated with avidin, by adding the substrate, when enzyme-conjugated avidin is used in Step (7);

(Step 9) stopping the enzyme reaction and the coloring reaction by adding a stop solution to the microplate; and,

(Step 10) measuring the colorimetric intensity, fluorescence intensity or luminescence intensity.

The one-pot method corresponds to the methods as described above from (22) to (24) of the present invention.

Specifically, the first is the immunoassay method comprising at least the following steps (a) and (b):

(a) reacting a sample with the fusion polypeptide-immobilized insoluble carrier of the present invention; and

(b) reacting the complex formed by the binding of the oxidized LDL in the sample to the fusion polypeptide-immobilized insoluble carrier, with an antibody labeled with a labeling agent capable of providing a detecting signal by itself or by reacting with another substance, said antibody having a reactivity to oxidized LDL or apolipoprotein B.

The second is an immunoassay method comprising at least the following steps (a) and (b):

(a) reacting a sample with an antibody labeled with a labeling agent capable of giving a detecting signal by itself or by reacting with another substance, said antibody having a reactivity to oxidized LDL or apolipoprotein B; and

(b) reacting the complex formed by the binding of the antibody to oxidized LDL in the sample, with the fusion polypeptide-immobilized insoluble carrier of the present invention.

The third is an immunoassay method comprising at least the following step (a);

(a) reacting a mixture comprising the fusion polypeptide-immobilized insoluble carrier of the present invention, an antibody labeled with a labeling agent capable of giving a detecting signal by itself or by reacting with another substance, said antibody having a reactivity to oxidized LDL or apolipoprotein B, and a test sample.

A specific example of assaying method of the present invention described in the first to third methods mentioned above is indicated below, in which the "insoluble carrier" are beads and the "labeling agent" is an enzyme such as peroxidase or biotin; the method comprises, for example, the steps as described below, but is not to be construed as being limited to the specific example.

The first method comprises the following steps;

(Step 1) preparing fusion polypeptide-immobilized beads, by immobilizing the fusion polypeptide of the present invention on beads;

(Step 2) reacting a sample with the fusion polypeptide immobilized on the beads by adding the beads and a sample such as human plasma together with a buffer solution into a container having internal spaces such as a test tube, microplate or tube;

(Step 3) removing the liquid content from the container and washing the beads;

(Step 4) preparing a labeled antibody by labeling an antibody having a reactivity to oxidized LDL or apolipoprotein B with biotin or an enzyme such as peroxidase;

(Step 5) reacting the labeled antibody with the complex formed through the reaction between a denatured LDL such as oxidized LDL in the sample and the fusion polypeptide immobilized on the beads by adding the labeled antibody to the container containing beads washed in Step 3;

(Step 6) removing the liquid content from the container and washing out the unreacted labeled antibody from the beads;
(Step 7) reacting the labeling agent moiety of the labeled antibody with a substrate selected depending on the type of the enzyme used (when the labeled antibody used in Step 5 is labeled with an enzyme such as peroxidase), avidin or enzyme-conjugated avidin (when the labeled antibody used in Step 5 is labeled with biotin), by adding, if necessary together with a coloring agent, the substrate, or avidin or enzyme-conjugated avidin to the container containing the beads washed in Step 6;
(Step 8) reacting the enzyme conjugated with avidin with a substrate for the enzyme selected depending on the type of the enzyme conjugated with avidin, by adding the substrate when enzyme-conjugated avidin is used in Step 7;
(Step 9) stopping the enzyme reaction and the coloring reaction by adding a stop solution to the reaction system of Step 7 or 8; and,
(Step 10) measuring the colorimetric intensity, fluorescence intensity or luminescence intensity.

The second method comprises steps such as the following.

(Step 1) preparing a labeled antibody by labeling an antibody having a reactivity to oxidized LDL or apolipoprotein B with biotin or an enzyme such as peroxidase;
(Step 2) reacting the labeled antibody with a sample such as human plasma by adding the labeled antibody and the sample together with a buffer solution into a container having internal spaces such as a test tube, microplate or tube.
(Step 3) preparing fusion polypeptide-immobilized beads by immobilizing the fusion polypeptide of the present invention on beads;
(Step 4) reacting the fusion polypeptide immobilized on the beads with the complex formed by reacting the labeled antibody with a denatured LDL such as oxidized LDL in the sample by adding the beads to the reaction system in Step 2;
(Step 5) removing the liquid content from the container and washing out the unreacted labeled antibody from the beads;
(Step 6) reacting the labeling agent moiety of the labeled antibody with a substrate selected depending on the type of the enzyme used (when the labeled antibody used in Step 2 is labeled with an enzyme such as peroxidase), avidin or enzyme-conjugated avidin (when the labeled antibody used in Step 2 is labeled with biotin), by adding, if necessary together with a coloring agent, the substrate, or avidin or enzyme-conjugated avidin to the container containing the beads washed in Step 5.;
(Step 7) reacting the enzyme conjugated with avidin with a substrate for the enzyme selected depending on the type of the enzyme, when enzyme-conjugated avidin is used in Step 6;
(Step 8) stopping the enzyme reaction and the coloring reaction by adding a stop solution to the reaction system in Step 6 or 7; and,
(Step 9) measuring the colorimetric intensity, fluorescence intensity or luminescence intensity.

The third method comprises of steps such as the following:

(Step 1) preparing fusion polypeptide-immobilized beads by immobilizing the fusion polypeptide of the present invention on beads;
(Step 2) preparing a labeled antibody by labeling an antibody having a reactivity to oxidized LDL or apolipoprotein B with biotin or an enzyme such as peroxidase;
(Step 3) simultaneously reacting the fusion polypeptide immobilized on the beads, the labeled antibody, and a sample such as human plasma by adding, together with a buffer solution, the fusion polypeptide immobilized-beads prepared in Step 1, the labeled antibody prepared in Step 2, and the sample into a container having internal spaces such as a test tube, plate, or tube.
(Step 4) removing the liquid content from the container and washing out the unreacted labeled antibody from the beads;
(Step 5) reacting the labeling agent moiety of the labeled antibody with a substrate selected depending on the type of the enzyme used (when the labeled antibody used in Step 3 is labeled with an enzyme such as peroxidase), avidin or enzyme-conjugated avidin (when the labeled antibody used in Step 3 is labeled with biotin), by adding, if necessary together with a coloring agent, the substrate, or avidin or enzyme-conjugated avidin to the container containing the beads washed in Step 4;
(Step 6) reacting the enzyme conjugated with avidin with a substrate for the enzyme selected depending on the type of the enzyme conjugated with avidin by adding the substrate, when enzyme-conjugated avidin is used in Step 5;

(Step 7) stopping the enzyme reaction and the coloring reaction by adding a stop solution to the reaction system in Step 5 or 6; and,

(Step 8) measuring the colorimetric intensity, fluorescence intensity or luminescence intensity.

The single antibody solid phase method corresponds to the method as described above in (25) of the present invention, and is specifically a method of immunoassay comprising at least the following step (a):

(a) reacting the fusion polypeptide-immobilized insoluble carrier of the present invention with a sample and a standard of oxidized LDL labeled with a labeling agent capable of providing a detectable signal by itself or by reacting with another substance.

A specific example of the assaying method of the present invention is indicated below, in which the "insoluble carrier" is a "multi-well microplate" and the "labeling agent" is an enzyme such as peroxidase or biotin; the method comprises, for example, the steps as described below, but the method is not to be construed as being restricted to the specific example.

(Step 1) preparing a fusion polypeptide-immobilized microplate by immobilizing the fusion polypeptide of the present invention on a multi-well microplate;

(Step 2) preparing a labeled oxidized LDL standard by labeling a denatured LDL such as the oxidized LDL, which is the ligand of oxidized LDL receptor, with biotin or an enzyme such as peroxidase;

(Step 3) reacting a sample such as human plasma and the labeled standard competitively with the fusion polypeptide immobilized on the microplate by adding the sample and the labeled standard to the microplate;

(Step 4) washing out the unreacted labeled standard from the microplate;

(Step 5) reacting the labeling agent moiety of the labeled standard with a substrate selected depending on the type of the enzyme used (when the labeled standard used in Step 3 is labeled with an enzyme such as peroxidase), avidin or enzyme-conjugated avidin (when the labeled standard used in Step 3 is labeled with biotin), by adding, if necessary together with a coloring agent, the substrate, or avidin or enzyme-conjugated avidin to the microplate washed in Step 4;

(Step 6) reacting the enzyme conjugated with avidin with a substrate for the enzyme selected depending on the type of the enzyme conjugated with avidin, by adding the substrate, when enzyme-conjugated avidin is used in Step 5;

(Step 7) stopping the enzyme reaction and the coloring reaction by adding a stop solution to the microplate; and,

(Step 8) measuring the colorimetric intensity, fluorescence intensity or luminescence intensity.

The "affinity chromatography" as referred to in the present invention indicates the method of separating or purifying a target substance in samples (for example, the body fluid samples such as a serum and plasma; culture supernatants; or the supernatant fluids obtained by centrifugation, and so on) by utilizing the interaction (affinity) between a pair of materials, for example, antigen and antibody, enzyme and substrate, or receptor and ligand.

The method of the present invention relates to a method for separating or purifying a denatured LDL such as oxidized LDL in samples (for example, the body fluid samples such as a serum and plasma; culture supernatants; or the supernatant fluids obtained by centrifugation and such) by the affinity of a receptor and its ligand, specifically, the affinity of oxidized LDL receptor and its ligand, a denatured LDL such as oxidized LDL; specifically indicates,

(1) a method for separating a denatured LDL such as oxidized LDL in samples by contacting the sample with the above-defined insoluble carriers, such as filters or membranes, on which the fusion polypeptide of the present invention is immobilized; and

(2) a method for separating or purifying a denatured LDL such as oxidized LDL in samples, by immobilizing, in a usual manner (immobilization by physical adsorption, cross-linking to the carrier polymer, trapping in the carrier matrix, non-covalent bonding, and such) the fusion polypeptide of the invention on the insoluble carriers such as cellulose carriers, agarose carriers, polyacrylamide carriers, dextran carriers, polystyrene carriers, polyvinyl alcohol carriers, poly(amino acid) carriers or porous silica carriers; by filling columns made of glass, plastics, or stainless, with said insoluble carriers; by loading and eluting samples (for example, the body fluid samples such as serum and plasma; culture supernatants; or the supernatant fluids obtained by centrifugation and so on) through columns (for example, cylindrical column). The method as described above in (2) is in particular designated as affinity column chromatography. Any of the insoluble carriers can be used as insoluble carriers for the affinity column chromatography, as far as the fusion polypeptide of the present invention can be immobilized on the carriers. Such carriers include, for example, commercially available carriers such as SEPHAROSE 2B, SEPHAROSE 4B, SEPHAROSE 6B, CNBR-ACTIVATED SEPHAROSE 4B, AH-SEPHAROSE 4B, CH-SEPHAROSE 4B, ACTIVATED CH-SEPHAROSE 4B, EPOXY-ACTIVATED SEPHAROSE 6B, ACTIVATED THIOL-SEPHAROSE 4B, SEPHADEX, CM-SEPHADEX, ECH-SEPHAROSE 4B, EAH-SEPHAROSE 4B, NHS-ACTIVATED SEPHAROSE, THIOPROPYL SEPHAROSE 6B, and such, all of which are supplied by Pharmacia; BIO-GEL A, CELLEX, CELLEX AE, CELLEX-CM, CELLEX PAB, BIO-GEL P, HYDRAZIDE BIO-GEL P, AMINOETHYL BIO-GEL P, BIO-GEL CM, AFFI-GEL 10, AFFI-GEL 15, AFFI-PREP 10, AFFI-GEL HZ, AFFI-PREP HZ, AFFI-GEL 102, CM BIO-GEL A, AFFI-GEL HEPARIN, AFFI-GEL 501, AFFI-GEL 601, and such, all of which are supplied by Bio-Rad; CHROMAGEL A, CHROMAGEL P, ENZAFIX P-HZ, ENZAFIX P-SH, ENZAFIX P-AB, and such, all of which are supplied by Wako Pure Chemical Industries Ltd.; AE-CELLULOSE, CM-CELLULOSE, PAB CELLULOSE and such, all of which are supplied by Serva.

The term "pharmaceutical composition" as referred to in the present invention comprises the fusion polypeptide of the present invention as an active ingredient, and one or more pharmaceutically acceptable carriers, such as, an excipients, a diluent, an expander, a disintegrating agent, a stabilizer, a preservative, a buffer, an emulsifier, an aromatic, a colorant, a sweetener, a viscosity increasing agent, a flavor, a solubilizing agent, or other additives, in dosage form of tablets, pills, powders, granules, injections, solutions, capsules, troches, elixirs, suspensions, emulsions, or syrups which can be administered orally or parenterally.

In particular, the injection can be produced by dissolving or suspending the fusion polypeptide in a non-toxic, pharmaceutically acceptable carrier such as physiological saline or commercially available distilled water for injection by adjusting the concentration to 0.1 μ g/ml carrier to 10 mg/ml carrier. The injection thus produced can be administered to a human patient in need of treatment in a dose of 1 μ g to 100 mg/kg body weight, preferably 50 μ g to 50 mg/kg body weight once or several times a day: Examples of administration route are medically appropriate administration routes such as intravenous injection, subcutaneous injection, intradermal injection, intramuscular injection, or intraperitoneal injection, preferably intravenous injection.

The pharmaceutical composition of the present invention is effective for preventing and treating various disorders such as arteriosclerosis and hyperlipidemia attributed to abnormal behavior of an oxidized-LDL receptor and/or a denatured LDL such as an oxidized LDL.

## Brief Description of the Drawings

Figure 1 schematically shows the structure of immunoglobulin (IgG).

Figure 2 schematically shows the linkage between a cDNA encoding an extracellular domain of bovine LOX-1 and a vector DNA in plasmid pBLOX-1-Fc.

Figure 3 shows the eletrophoretic pattern of the recombinant fusion polypeptide (bLOX-1-Fc) in Western blot analysis.

Figure 4 shows the calibration curve of rabbit-derived oxidized LDL standard measured by the quantification method of the present invention.

Figure 5 shows the calibration curve of human-derived oxidized LDL standard measured by the quantification method of the present invention.

Figure 6 shows the oxidized LDL amount in the plasma of normal rabbits and hyperlipidemic model rabbits measured by the quantification technique of the present invention.

Figure 7 shows the calibration curve of rabbit-derived oxidized LDL standard measured by the quantification method of the present invention.

Figure 8 shows the calibration curve of human-derived oxidized LDL standard measured by the quantification method of the present invention.

Figure 9 shows the denatured LDL amount in serum of healthy volunteer and the patients suffering from hyperlipidemia, measured by the quantification method of the present invention.

## Best Mode for Carrying out the Invention

The present invention is illustrated in detail below with reference to working examples, but is not to be construed as being limited thereto.

### Example 1: Preparation of the fusion polypeptide

The cDNA encoding the bovine oxidized-LDL receptor LOX-1 (bLOX-1) (SEQ ID NO: 6) was prepared in the same manner as described in the previous reports (Nature, Vol. 386, p73-77, 1997 and JP-A No. Hei 9-98787).

The obtained cDNA was amplified by PCR using a pair of primers [5'-GGGGATCCTGATCTCATAAAGAAACAG-3' (SEQ ID NO: 8) and 5'-GCGGATCCTGTGCTCTCAATAGATTCGC-3' (SEQ ID NO: 9)] to prepare a cDNA fragment comprising the cDNA encoding the extracellular domain of the bovine LOX-1 (nucleotides 215 to 844 of SEQ ID NO: 6) in which *Bam*HI cleavage site was added to the ends.

Plasmid pCd51neg1 comprising genomic DNA with the exons encoding each of the hinge region, C $\gamma_1$2 and C $\gamma_1$3 of human IgG1 (refer to DNA and Cell Biol., Vol. 9, p347-353, 1990; obtained from Dr. B. Seed of Massachusetts General Hospital; comprising the nucleotide sequence of SEQ ID NO: 7) was linearized by digestion with *Bam*HI.

The cDNA encoding the extracellular domain of the bovine LOX-1, obtained in the manner described above, was linked to the *Bam*HI cleavage sites (nucleotide 169 of SEQ ID NO: 7) in the linearized plasmid using T4 DNA ligase to construct plasmid pBLOX-1-Fc (Fig. 2).

Subconfluent monolayer CHO-K1 cells cultured in HamF12 medium with 10% fetal bovine serum (FBS) were co-transfected by pBLOX-1-Fc (1 μ g) and the expression vector pSVbsr [10 ng, manufactured by Funakoshi; including blasticidin S-resistance (bsr) gene and a promoter derived from SV40 virus] using lipofectamine (GIBCO).

After culturing for 48 hours, the medium was replaced by HamF12 medium with blasticidin-S (10 μ g/ml, Funakoshi) for further culturing to select and obtain the transformants co-transfected with pBLOX-1-Fc and pSVbsr.

The obtained transformants were maintained in the HamF12 medium with 10 % fetal calf serum (FCS) and blasticidin-S (10 μ g/ml, Funakoshi).

For purifying bLOX-1-Fc, the medium of the confluent transformant CHO-K1 cells cultured in the HamF12 medium with blasticidin-S (10 μ g/ml, Funakoshi) was replaced by CHO-SFM-11 (GIBCO/BRL) and cultured for three days. This process was repeated three times to obtain 800 ml of culture supernatant. The bLOX-1-Fc in the culture supernatant was purified using AFFI-GEL PROTEIN A MAPS-II KIT (Bio-rad) in the following manner.

The culture supernatant was added onto a protein A agarose gel column previously equilibrated with the binding buffer. The column was washed with the binding buffer (15 bed volume), and elution was performed with the elution buffer (5 bed volume). The eluate was collected -and dialyzed by replacing the dialysis solution by phosphate buffer more than once to obtain purified bLOX- 1-Fc. The obtained purified bLOX-1-Fc was ultrafiltered using CENTRIPREP (Amicon) for concentration. Using BCA PROTEIN ASSAY KIT (PIERCE), 866 μ g/ml purified bLOX-1-Fc was confirmed.

The above purified bLOX- 1-Fc was also confirmed by Western blot analysis described below.

Purified bLOX-1-Fc was applied to 12.5% SDS agarose gel (Daiichi Chemical), electrophoresed, and blotted onto IMMOBILON MEMBRANE (Millipore). The membrane was blocked with BLOCK ACE (Snow Brand) overnight. The reaction was conducted using the biotin labeled goat anti-human IgG antibody as the primary antibody and ABC KIT (Vector), and stained using KONICA IMMUNOSTAIN KIT (Fig. 3).

SEQ ID NOs: 3 and 4 show the amino acid sequence and the cDNA sequence of bLOX-1-Fc, respectively.

Example 2: Preparation of the fusion polypeptide-immobilized microplate

Purified bLOX-1-Fc prepared in Example 1 was diluted with phosphate buffer into 5 μ g/ml. The diluted solution was seeded into each well of a 96-well microplate (Nunc) and incubated at 4°C overnight to absorb bLOX-1-Fc onto the microplate. Each well was washed with phosphate buffer twice. The washing solution was discarded and the blocking agent [320 μ l, 25% (v/v), BLOCK ACE, Dainippon pharmaceuticals] was added to each well and incubated at room temperature for 6 hours to block the sites where bLOX-1-Fc was not bound. Each well was washed with phosphate buffer three times to prepare the bLOX-1-Fc-immobilized microplate.

Example 3: Preparation of human oxidized LDL standard

Plasma of the healthy volunteer was given with potassium bromide (Kbr), specific gravity adjusted to 1.019, and centrifuged by BECKMAN L-80 ultracentrifuge for 20 hours at 58,000 rpm to collect the lower layer in another tube. The collected amount was measured. The collected solution was adjusted at a specific gravity of 1.063 by adding potassium bromide, and centrifuged by BECKMAN L-80 ultracentrifuge for 20 hours at 58,000 rpm. The upper layer was collected in another tube. The collected fraction was dialyzed against phosphate buffer (the buffer was replaced more than once) to obtain the purified human LDL. Protein amount was measured using BCA PROTEIN ASSAY KIT (PIERCE). Protein amount was 10.3 mg/ml.

For preparing an oxidized LDL from the obtained purified LDL, the mixture of the purified LDL and copper sulfate ($CuSO_4$) 'adjusted at 3 mg/ml and 75 μ M, respectively, was incubated in a $CO_2$ incubator for 20 hours, and dialyzed against 0.15 M sodium chloride containing EDTA (the dialysis solution was replaced more than once) to obtain human oxidized LDL. Protein amount was measured by BCA PROTEIN ASSAY KIT (PIERCE). Protein amount was 2.32 mg/ml.

Each of purified LDL and oxidized LDL prepared in the above manner was loaded on an agarose gel (TITAN GEL LIPOPROTEINS, Helena Laboratory) and electrophoresed (constant voltage: 90 volt, for 25 min). The gel was dried in a drier at 55°C. Lipid was stained with FAT RED 7B stain and destained with 70 % ethanol. The gel was dried again in the drier at 55°C. Using TBARS (lipid peroxide LPO) measurement kit (LPO test Wako, Wako Pure Chemicals), oxidation degree of the lipid was measured. The oxidation degree of the obtained lipid was 24.74 mol/mg protein. The human oxidized LDL obtained in this manner was used as a standard.

Example 4: Preparation of the standard for rabbit oxidized LDL

Plasma of Japanese white rabbits was adjusted to a specific gravity of 1.019 by adding potassium chloride (KBr), and centrifuged by BECKMAN L-80 ultracentrifuge for 20 hours at 58,000 rpm to collect the lower layer in another tube. The collected amount was measured and the specific gravity was adjusted to 1.063 by adding potassium bromide, and centrifuged by BECKMAN L-80 ultracentrifuge for 20 hours at 58,000 rpm. The upper layer was collected in another tube. The collected fraction was dialyzed against phosphate buffer (the buffer was replaced more than once) to obtain the purified rabbit LDL. Protein amount was measured using BCA PROTEIN ASSAY KIT (PIERCE). The protein amount was 895.6 μ g/ml.

To prepare oxidized LDL from the obtained purified LDL, a mixture of purified LDL and copper sulfate ($CuSO_4$) adjusted at 100 μ g/ml and 75 μ M, respectively, was incubated in a $CO_2$ incubator for 20 hours, dialyzed against 0.15 M sodium chloride containing EDTA (the dialysis solution was replaced more than once) to obtain rabbit oxidized LDL. Protein amount was measured by BCA PROTEIN ASSAY KIT (PIERCE). The protein amount was 307.1 μ g/ml. Rabbit oxidized LDL obtained in this manner was used as a standard.

Example 5: Preparation of calibration curves

Experiment 1

The standard of purified human oxidized LDL prepared in Example 3 was diluted to various concentrations (500, 250, 125, 62.5, 31.25, 15.625, and 7.8125 ng/ml) with phosphate buffer containing 20% bovine newborn serum (GIBCO), added to each well of the bLOX-1-Fc immobilized microplate prepared in Example 2, and incubated at 4°C for 24 hours.

Similarly, the standard of the purified rabbit oxidized-LDL prepared in Example 4 was diluted to various concentrations (10, 5, 2.5, 1.25, 0.625, 0.3125, and 0.15625 μ g/ml) with phosphate buffer containing 20% bovine newborn serum (GIBCO), added to each well of the bLOX-1-Fc immobilized microplate prepared in Example 2, and incubated at 4°C for 24 hours.

Each plate was washed with phosphate buffer three times. Peroxidase labeled sheep anti-human apolipoprotein B antibody (100 μ 1, Funakoshi, Code No: PP086) diluted with 1% bovine serum albumin (BSA) into 1/1000 was added into each well, and incubated at room temperature for 2 hours. The plates were washed with phosphate buffer 6 times, and ortho-phenylene diamine (100 μ l of 100 μ g/ml) dissolved in 0.1 M sodium citrate buffer (pH 5.5) and 0.02% hydrogen peroxide were added to each well, and incubated at room temperature. After 20 minutes, 2 M sulfuric acid (50 μ l) was added to each well to stop the reaction. Enzyme activity was determined by measuring fluorescence intensity at wave length 490 nm by a 96-well plate reader to plot calibration curve.

Figures 4 and 5 show calibration curves when using the rabbit oxidized LDL standard and the human oxidized LDL standard, respectively.

In the case of human oxidized LDL, a linear calibration curve was obtained at the extremely low concentration range of at least about 7.8125 ng/ml to 500 ng/ml (correlation coefficient: r =0.997).

In the case of rabbit oxidized LDL, a linear calibration curve was obtained at the extremely low concentration range of at least about 0.15625 μ g/ml to 10 μ g/ml (correlation coefficient: r = 0.996).

Experiment 2:

Calibration curves were plotted in the same manner as in Experiment 1 by further expanding the ranges of the standard dilution concentrations for purified human oxidized LDL and purified rabbit oxidized LDL, as follows.

(Human oxidized LDL)

2000, 1000, 500, 250, 125, 62.5, 31.25, 15.625, 7.8125, and 3.90625 ng/ml

(Rabbit oxidized LDL)

40, 20, 10, 5, 2.5, 1.25, 0.625, 0.3125, and 0.15625 μ g/ml

Figures 7 and 8 show the calibration curves for rabbit oxidized LDL standard and for human oxidized LDL standard, respectively.

In the case of human oxidized LDL, at the extremely low concentration range of at least about 3.90625 ng/ml to 1000 ng/ml, a linear calibration curve was obtained (correlation coefficient: r = 0.996).

In the case of rabbit oxidized LDL, at the extremely low concentration at the range of at least about 0.15625 μ g/ml to 10 μ g/ml, a linear calibration curve was obtained (correlation coefficient: r = 0.992).

Example 6: Quantification of rabbit oxidized LDL

Each plasma collected from normal Japanese white rabbits (17 individuals) and hyperlipidemic model rabbits [Watanabe Heritable Hyperlipidemic Rabbit (WHHL), 12 individuals] was diluted with phosphate buffer containing 20% bovine newborn serum (GIBCO). Each diluted plasma (100 μ l) was added to each well of the bLOX-1-Fc immobilized microplate prepared in Example 2, and incubated at 4°C for 24 hours. The plates were washed three times with phosphate buffer. Peroxidase labeled sheep anti-human apolipoprotein B antibody (100 μ l, Funakoshi: Code NO: PP086) diluted with 1% bovine serum albumin (BSA) into 1/1000 was added to each well, and incubated at room temperature for 2 hours. The plates were washed 6 times with phosphate buffer. Ortho-phenylene diamine (100 μ l of 100 μ g/ml) dissolved in 0.1 M sodium citrate buffer (pH 5.5) and 0.02% hydrogen peroxide were added to each well and incubated at room temperature. After 20 minutes, 2 M sulfuric acid (50 μ l) was added to each well to stop the reaction. Fluorescence intensity at wave length 490 nm was measured with a 96-well microplate reader to determine enzyme activity and quantify oxidized LDL in plasma (Figure 6).

The concentration of oxidized LDL in plasma of the normal rabbits was significantly low, while that in the plasma of the hyperlipidemic rabbits was significantly higher than that in the plasma of the normal rabbits. The quantification sensitivity (detection sensitivity) for rabbit oxidized LDL in the present invention was confirmed to be extremely high.

Example 7: Quantification of human oxidized LDL

Heparinized blood was collected from veins of each of patients suffering from hyperlipidemia (more than 20 patients) and healthy volunteers (more than 20 individuals) who did not show any clinical symptoms of hyperlipidemia at the time of collection. Serum was separated by centrifuging the blood (about 3,000 rpm, 20 min). The obtained serum was immediately frozen and stored at -80°C. The average age of the subjects was about 57.5 (youngest: aged 40, eldest: aged 70).

The frozen serum was thawed in a refrigerator, diluted to 1/10 with phosphate buffer, added to each well of the bLOX-1-Fc immobilized microplate prepared in Example 2, and incubated at 4°C for 24 hours.

The plates were washed three times with phosphate buffer. Peroxidase labeled sheep anti-human apolipoprotein B antibody (100 μ l, Funakoshi, Code No: PP086) diluted into 1/1000 with 1% bovine serum albumin (BSA) to each well, and incubated at room temperature for 2 hours. The plates were washed 6 times with phosphate buffer. Ortho-phenylene diamine (100 μ l of 100 μ g/ml) dissolved in 0.1 M sodium citrate buffer (pH 5.5) and 0.02 % hydrogen peroxide were added to each well, and incubated at room temperature. After 20 minutes, 2M sulfuric acid (50 μ l) was added to each well to stop the reaction. Fluorescence intensity at wave length 490 nm was measured by a 96-well plate reader to determine enzyme activity and quantify oxidized LDL in serum (Fig. 9).

The average amount of denatured LDL (LOX-1 ligand) in the serum of patients suffering from hyperlipidemia was about 1.6 times higher than that of healthy volunteers. It was confirmed that the amount of denatured LDL (LOX-1 ligand) is significantly increased in the patients suffering from hyperlipidemia.

Example 8: Preparation of a pharmaceutical composition and oxidized LDL inhibiting activity

To use as an injection, purified bLOX-1-Fc (1-200 μ g/ml) prepared in Example 1 was added to distilled water for injections (10 ml).

This injection is intravenously administered to hyperlipidemic model rabbits (WHHL) or arteriosclerotic model rabbits at 1-10 mg/kg (the first administration: 0 hour), and given again every 10 to 30 hours at the same concentration. The plasma is regularly collected after each administration and the amount of oxidized LDL in each plasma sample is measured in the same manner as in Example 6. bLOX-1-Fc would lower the amount of oxidized LDL in the blood of rabbits.

Industrial Applicability

The fusion polypeptide of the present invention, specifically the fusion polypeptide composed of the extracellular domain of oxidized-LDL receptor (for example, human LOX-1) and a portion of a constant region on immunoglobulin heavy chain (for example, Fc of human IgG), is useful not only as a component in the assay for detecting and quantifying a denatured LDL such as oxidized LDL in body fluids (for example, serum, plasma, and such) of mammals (for example, a healthy person, a patient, and such), but also as a component in separation and purification of a denatured LDL such as the oxidized LDL.

By using the fusion protein of the present invention, a denatured LDL, such as oxidized LDL existing in the body fluids of the patients suffering from arteriosclerosis, hyperlipidemia, and so on can be conveniently and highly sensitively detected and quantified in an intact condition, and a clinically applicable quantification and detection method and a kit used for the method can be provided.

The detection and quantification method and the kit of the present invention are extremely useful for diagnosing various diseases such as arteriosclerosis and hyperlipidemia.

Moreover, as the fusion polypeptide of the present invention comprises a portion of a constant region in an immunoglobulin such as IgG (for example, Fc) as a fusion partner, the fusion polypeptide can be extremely conveniently purified by using affinity column chromatography using the characteristics of protein A that specifically binds to fragments of the immunoglobulin. Further, as various antibodies against Fc of various immunoglobulins have been provided, the immunoassay for the fusion polypeptide can be conveniently conducted using an antibody against the Fc.

The fusion polypeptide of the present invention is extremely useful not only as a tool for assaying (detection, quantification, and such), separating and purifying a denatured LDL such as the oxidized LDL described above, but also as an effective ingredient of pharmaceuticals by itself for preventing and treating diseases such as arteriosclerosis and hyperlipidemia.

## Claims

1. A fusion polypeptide comprising an extracellular domain of a mammalian oxidized-LDL receptor and a portion of a mammalian immunoglobulin (Ig) heavy chain.

2. The fusion polypeptide of claim 1, wherein the mammalian immunoglobulin is a human immunoglobulin.

3. The fusion polypeptide of claim 1 or 2, wherein the immunoglobulin is IgG.

4. The fusion polypeptide of any one of claims 1 to 3, wherein the portion of a immunoglobulin heavy chain is a constant region or a portion of constant region of an immunoglobulin heavy chain.

5. The fusion polypeptide of claim 4, wherein the portion of a constant region is composed of a hinge region, C2 domain and C3 domain of IgG, IgA or IgD.

6. The fusion polypeptide of claim 4, wherein a portion of a constant region is composed of C2 domain, C3 domain, and C4 domain of IgM or IgE.

7. The fusion polypeptide of claim 1, wherein the portion of a heavy chain in the mammalian immunoglobulin is composed of a hinge region, C2 domain and C3 domain of human IgG.

8. The fusion polypeptide of any one of claims 1 to 7, wherein the mammalian oxidized-LDL receptor is a human oxidized-LDL receptor.

9. The fusion polypeptide of claim 8, wherein the human oxidized-LDL receptor is a polypeptide comprising an amino acid sequence identical or substantially identical to the amino acid sequence of SEQ ID NO: 1.

10. The fusion polypeptide of any one of claims 1 to 7, wherein the mammalian oxidized-LDL receptor is a bovine oxidized-LDL receptor.

**11.** The fusion polypeptide of claim 10, wherein the bovine oxidized-LDL receptor is a polypeptide comprising an amino acid sequence identical or substantially identical to the amino acid sequence of SEQ ID NO: 2.

**12.** A fusion polypeptide comprising an amino acid sequence identical or substantially identical to the amino acid sequence of SEQ ID NO: 3.

**13.** A DNA encoding the fusion polypeptide of any one of claims 1 to 12.

**14.** A DNA comprising the nucleotide sequence of SEQ ID NO: 4 or 10.

**15.** A vector comprising the DNA of claim 13 or 14.

**16.** A transformant transfected by the vector of claim 15.

**17.** A fusion polypeptide-immobilized insoluble carrier, wherein the fusion polypeptide of any one of claims 1 to 12 is immobilized on an insoluble carrier.

**18.** The fusion polypeptide-immobilized insoluble carrier of claim 17, wherein the insoluble carrier is selected from the group consisting of plates, test tubes, beads, balls, filters, and membranes.

**19.** The fusion polypeptide-immobilized insoluble carrier of claim 17, wherein the insoluble carrier is a filter or a membrane, or one used for an affinity column chromatography.

**20.** A kit used for detecting or quantifying an oxidized LDL, comprising the fusion polypeptide-immobilized insoluble carrier of claim 17 or 18, or the fusion polypeptide of any one of claims 1 to 12.

**21.** A method for detecting or quantifying an oxidized LDL by an immunoassay using the fusion polypeptide-immobilized insoluble carrier of claim 17 or 18, or the fusion polypeptide of any one of claims 1 to 12.

**22.** The method for detecting or quantifying an oxidized LDL by the immunoassay of claim 21, comprising at least following steps of (a) and (b);

(a) reacting a sample with the fusion polypeptide-immobilized insoluble carrier of claim 17 or 18, and,
(b) reacting the complex formed by binding an oxidized LDL in the sample to the fusion polypeptide-immobilized insoluble carrier, with an antibody labeled with a labeling agent capable of providing a detectable signal by itself or by reacting with another substance, said antibody having a reactivity to an oxidized LDL or apolipoprotein B.

**23.** The method for detecting or quantifying an oxidized LDL by an immunoassay of claim 21, comprising at least the following steps of (a) and (b);

(a) reacting a sample with an antibody labeled with a labeling agent capable of providing a detectable signal by itself or by reacting with another substance, said antibody having a reactivity to an oxidized LDL or apolipoprotein B, and
(b) reacting the complex formed by the binding between the antibody and an oxidized LDL in the sample, with the fusion polypeptide-immobilized insoluble carrier of claim 17 or 18.

**24.** A method for detecting or quantifying an oxidized LDL by an immunoassay comprising at least the following step of (a);

(a) reacting a mixture comprising the fusion polypeptide-immobilized insoluble carrier of claim 17 or 18, an antibody labeled with a labeling agent capable of providing a detectable signal by itself or by reacting with another substance, said antibody having a reactivity to an oxidized LDL or apolipoprotein B, and a sample.

**25.** The method for detecting or quantifying an oxidized LDL by an immunoassay of claim 21, comprising at least the following step of (a);

(a) reacting a fusion polypeptide-immobilized insoluble carrier of claim 17 or 18, with a sample and an oxidized LDL standard labeled with a labeling agent capable of providing a detectable signal by itself or by reacting with another substance, said antibody having a reactivity to an oxidized-LDL or apolipoprotein B.

**26.** A kit used for separating or purifying an oxidized LDL, comprising the fusion polypeptide-immobilized insoluble carrier of claim 17 or 19.

**27.** A method for separating or purifying an oxidized LDL, by affinity chromatography using the fusion polypeptide-immobilized insoluble carrier of claim 17 or 19.

**28.** A method for purifying the oxidized LDL of claim 27, wherein the affinity chromatography is an affinity column chromatography.

**29.** A pharmaceutical composition comprising a fusion polypeptide comprising an extracellular domain of a human oxidized-LDL receptor comprising the amino acid sequence of SEQ ID NO: 1, and a portion of a human immunoglobulin heavy chain, and a pharmaceutically acceptable carrier.

**30.** The pharmaceutical composition of claim 29, wherein the portion of a immunoglobulin heavy chain is a constant region or a portion of the constant region of an immunoglobulin heavy chain,

**31.** The pharmaceutical composition of claim 29 or 30, used for preventing or treating arteriosclerosis or hyperlipidemia.

Fig. 1

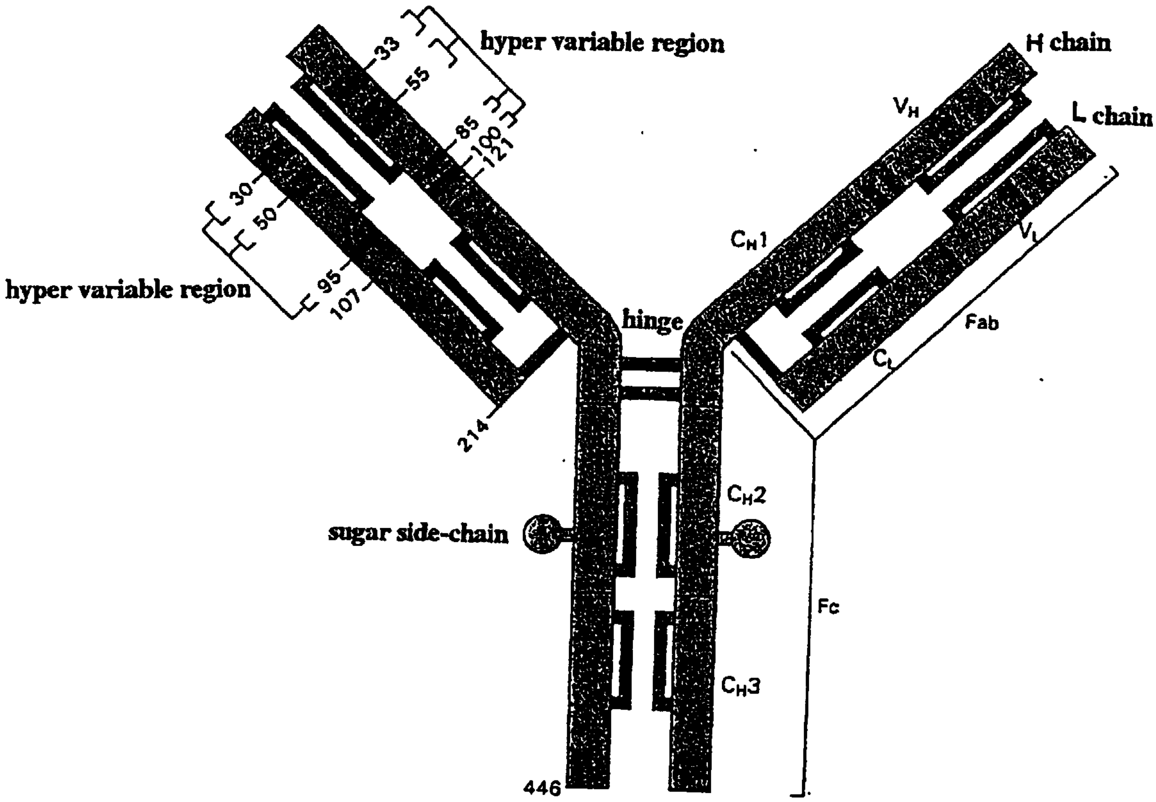
hyper variable region
33
55
85
100
121
30
50
95
107
hyper variable region
214
hinge
H chain
L chain
V_H
C_H1
V_L
Fab
C_L
C_H2
sugar side-chain
Fc
C_H3
446

Fig. 2

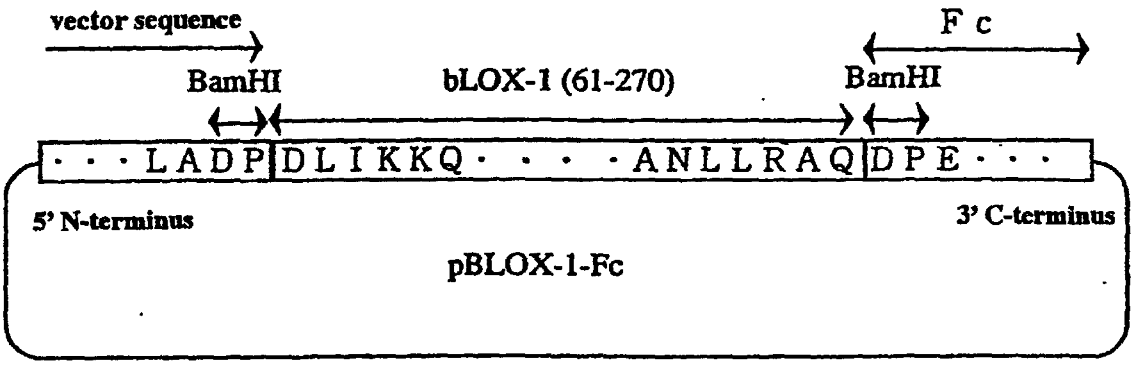
vector sequence
F c
BamHI
bLOX-1 (61-270)
BamHI
· · · L A D P D L I K K Q · · · · A N L L R A Q D P E · · ·
5' N-terminus
3' C-terminus
pBLOX-1-Fc

Fig. 3

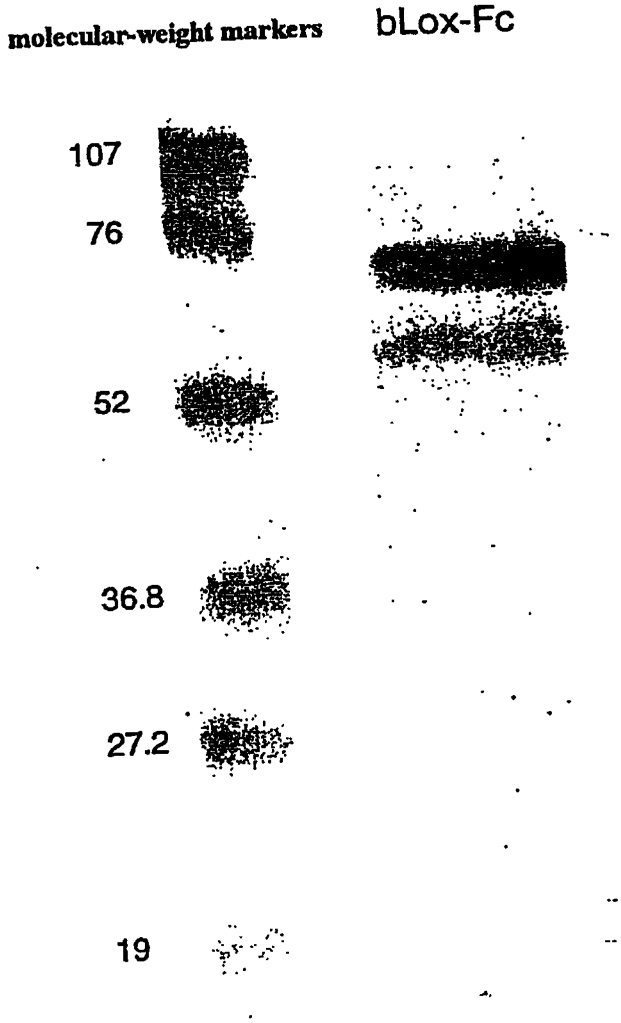

Fig. 4

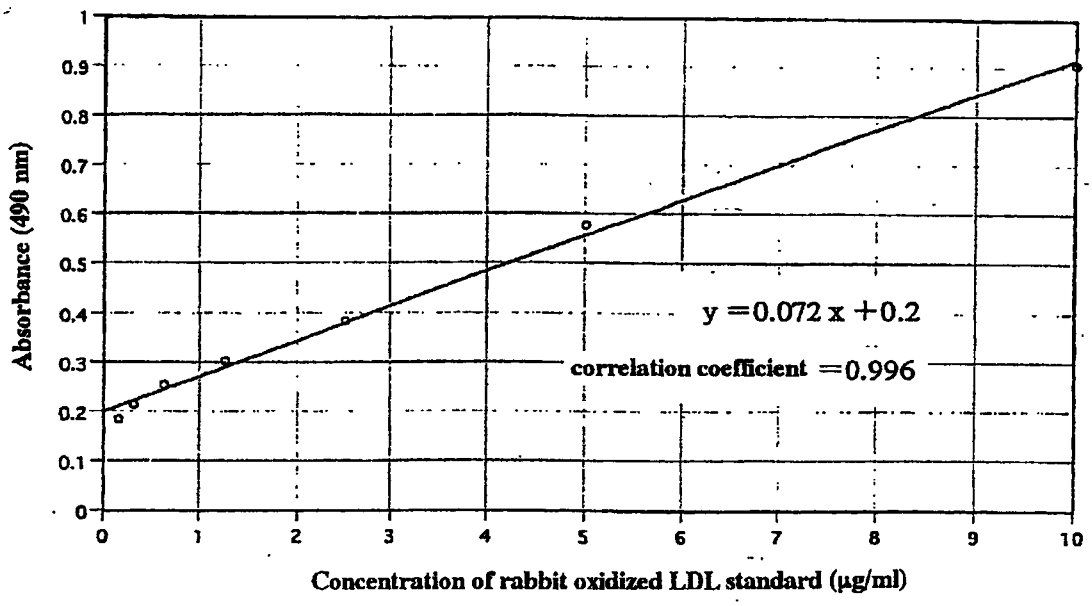
Absorbance (490 nm)
1
0.9
0.8
0.7
0.6
0.5
0.4
0.3
0.2
0.1
0
0 1 2 3 4 5 6 7 8 9 10
y =0.072 x +0.2
correlation coefficient =0.996
Concentration of rabbit oxidized LDL standard (µg/ml)

Fig. 5

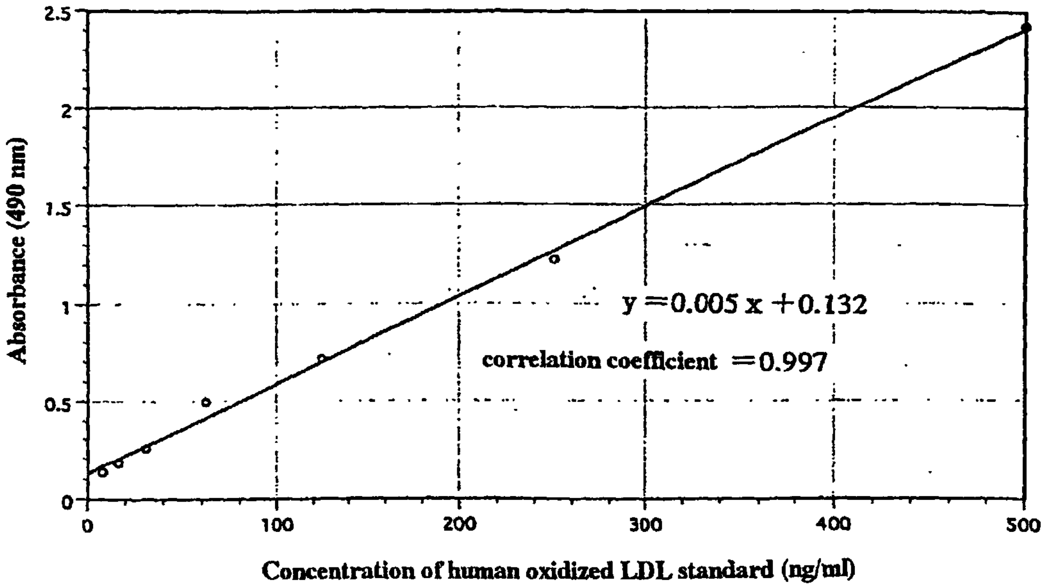
2.5
2
1.5
1
0.5
0
Absorbance (490 nm)
0
100
200
300
400
500
Concentration of human oxidized LDL standard (ng/ml)
y =0.005 x +0.132
correlation coefficient =0.997

Fig. 6

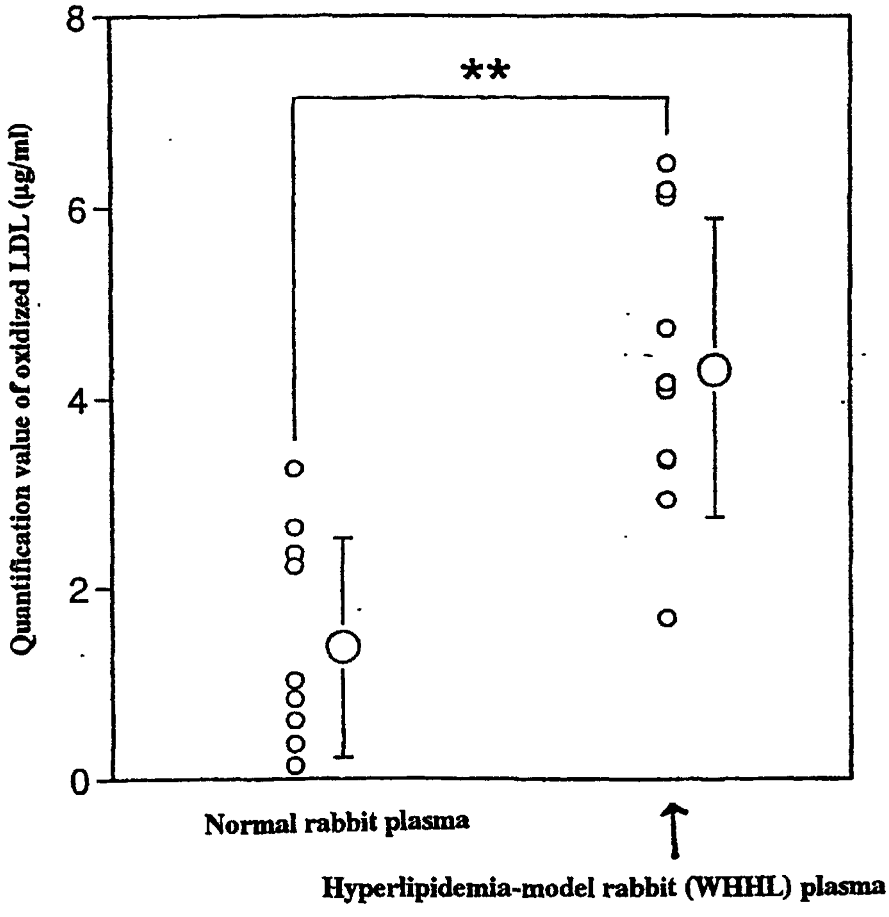
8
6
4
2
0
**
Quantification value of oxidized LDL (µg/ml)
Normal rabbit plasma
Hyperlipidemia-model rabbit (WHHL) plasma

** : P<0.01

Fig. 7

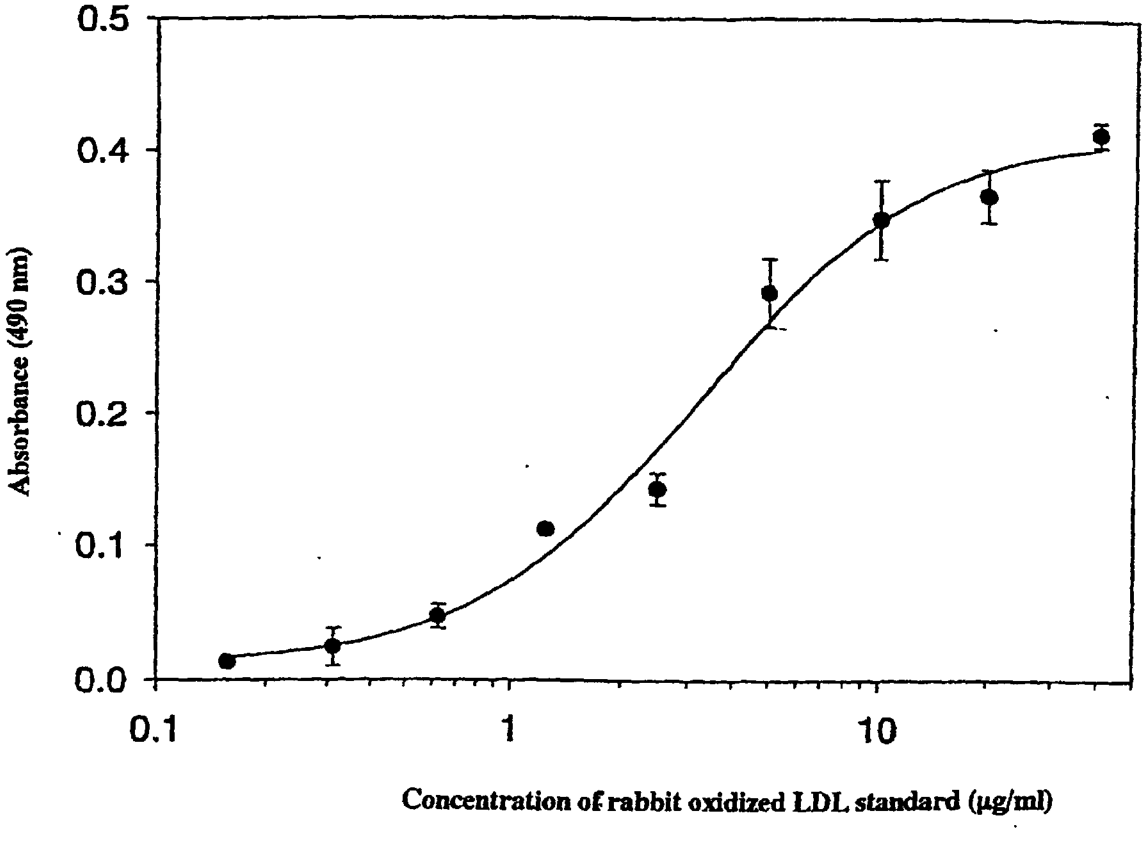
0.5
0.4
0.3
0.2
0.1
0.0
Absorbance (490 nm)
0.1
1
10
Concentration of rabbit oxidized LDL standard (μg/ml)

Fig. 8

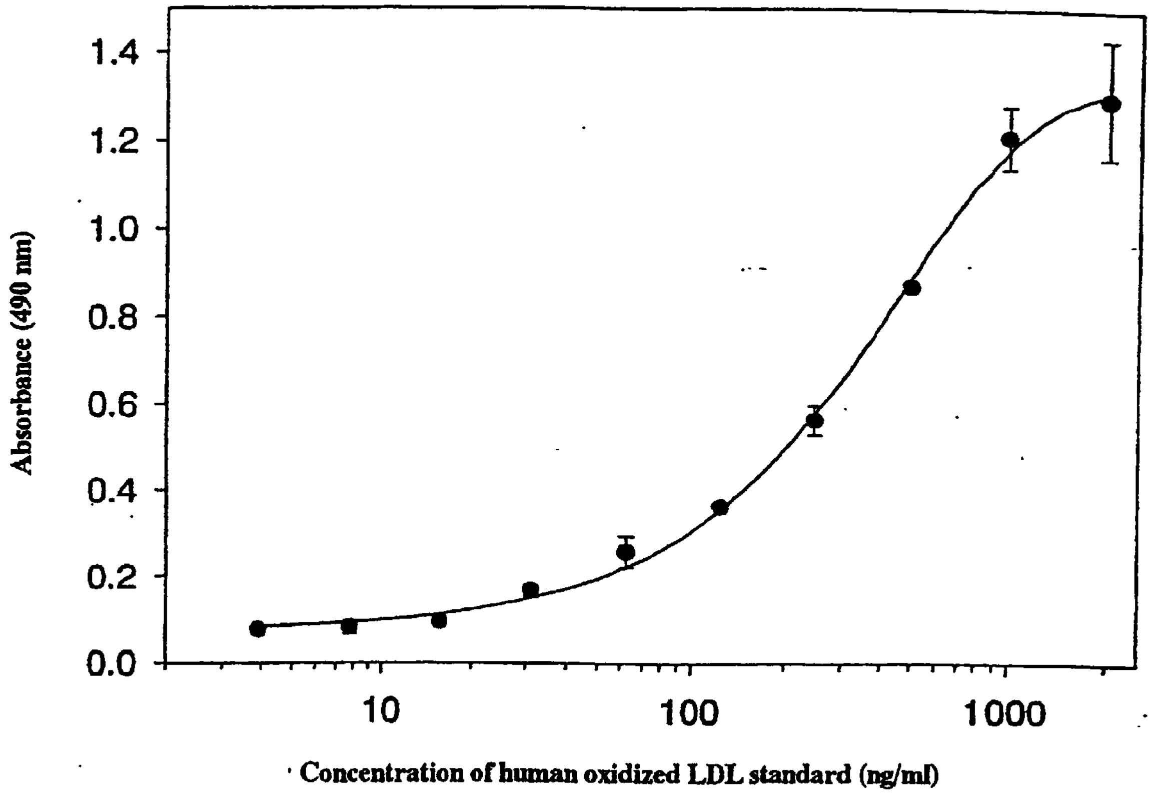
1.4
1.2
1.0
0.8
0.6
0.4
0.2
0.0
Absorbance (490 nm)
10
100
1000
Concentration of human oxidized LDL standard (ng/ml)

Fig. 9

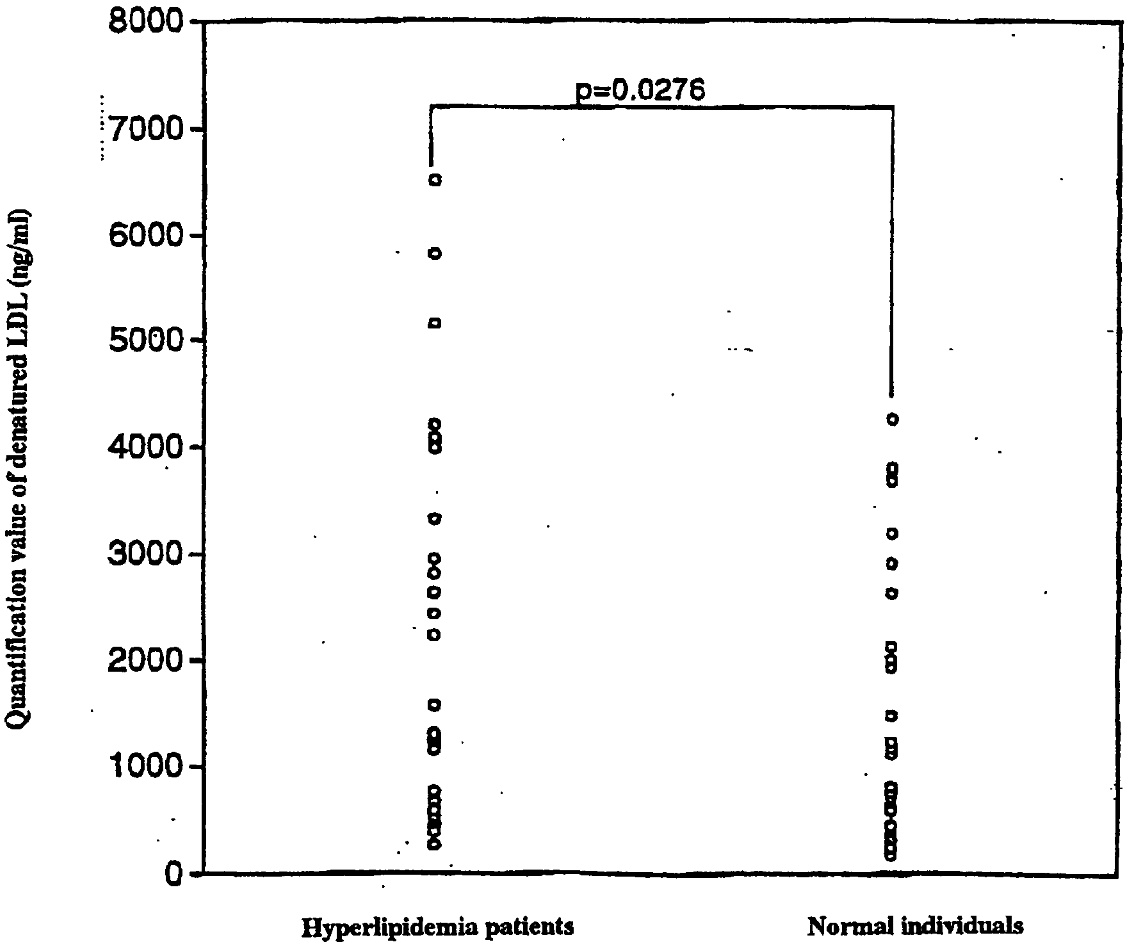
p=0.0276
Quantification value of denatured LDL (ng/ml)
8000
7000
6000
5000
4000
3000
2000
1000
0
Hyperlipidemia patients
Normal individuals

## INTERNATIONAL SEARCH REPORT

International application No. PCT/JP98/05744

A. CLASSIFICATION OF SUBJECT MATTER
Int.Cl⁶ C07K19/00, C07K14/705, C12N15/62, C07K17/00, G01N33/53, A61K38/17

According to International Patent Classification (IPC) or to both national classification and IPC

B. FIELDS SEARCHED

Minimum documentation searched (classification system followed by classification symbols)
Int.Cl⁶ C07K19/00, C07K14/705, C12N15/62, C07K17/00, G01N33/53, A61K38/17

Documentation searched other than minimum documentation to the extent that such documents are included in the fields searched

Electronic data base consulted during the international search (name of data base and, where practicable, search terms used)
BIOSIS PREVIEWS (DIALOG), WPI (DIALOG)

C. DOCUMENTS CONSIDERED TO BE RELEVANT

| Category* | Citation of document, with indication, where appropriate, of the relevant passages | Relevant to claim No. |
|---|---|---|
| Y | JP, 9-98787, A (Nippon Chemiphar Co., Ltd.), 15 April, 1997 (15. 04. 97) & WO, 96/17058, A & EP, 795605, A | 1-31 |
| Y | JP, 6-160395, A (Behringwerke AG.), 7 June, 1994 (07. 06. 94) & EP, 488170, A & US, 5639597, A | 1-31 |
| Y | JP, 5-247094, A (Behringwerke AG.), 24 September, 1993 (24. 09. 93) & EP, 464533, A | 1-31 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 9 February, 1999 (09. 02. 99) | 16 February, 1999 (16. 02. 99) |
| Name and mailing address of the ISA/ Japanese Patent Office | Authorized officer |
| Facsimile No. | Telephone No. |

Form PCT/ISA/210 (second sheet) (July 1992)

SEQUENCE LISTING

<110> Japan Tobacco, Inc.

<120> Method for Quantifying Denatured LDL

<130> J1-010PCT

<140>
<141>

<150> JP P1997-364981
<151> 1997-12-19

<150> JP P1998-349648
<151> 1998-12-09

<150> JP P1998-358170
<151> 1998-12-16

<160> 10

<170> PatentIn Ver. 2.0

<210> 1
<211> 273
<212> PRT
<213> Homo sapiens

<400> 1

```
Met Thr Phe Asp Asp Leu Lys Ile Gln Thr Val Lys Asp Gln Pro Asp
  1               5                  10                  15

Glu Lys Ser Asn Gly Lys Lys Ala Lys Gly Leu Gln Phe Leu Tyr Ser
             20                  25                  30
```

Pro Trp Trp Cys Leu Ala Ala Ala Thr Leu Gly Val Leu Cys Leu Gly
35 40 45

Leu Val Val Thr Leu Met Val Leu Gly Met Gln Leu Ser Gln Val Ser
50 55 60

Asp Leu Leu Thr Gln Glu Gln Ala Asn Leu Thr His Gln Lys Lys Lys
65 70 75 80

Leu Glu Gly Gln Ile Ser Ala Arg Gln Gln Ala Glu Glu Ala Ser Gln
85 90 95

Glu Ser Glu Asn Glu Leu Lys Glu Met Ile Glu Thr Leu Ala Arg Lys
100 105 110

Leu Asn Glu Lys Ser Lys Glu Gln Met Glu Leu His His Gln Asn Leu
115 120 125

Asn Leu Gln Glu Thr Leu Lys Arg Val Ala Asn Cys Ser Ala Pro Cys
130 135 140

Pro Gln Asp Trp Ile Trp His Gly Glu Asn Cys Tyr Leu Phe Ser Ser
145 150 155 160

Gly Ser Phe Asn Trp Glu Lys Ser Gln Glu Lys Cys Leu Ser Leu Asp
165 170 175

Ala Lys Leu Leu Lys Ile Asn Ser Thr Ala Asp Leu Asp Phe Ile Gln
180 185 190

Gln Ala Ile Ser Tyr Ser Ser Phe Pro Phe Trp Met Gly Leu Ser Arg
195 200 205

Arg Asn Pro Ser Tyr Pro Trp Leu Trp Glu Asp Gly Ser Pro Leu Met

210 215 220

Pro His Leu Phe Arg Val Arg Gly Ala Val Ser Gln Thr Tyr Pro Ser
225 230 235 240

Gly Thr Cys Ala Tyr Ile Gln Arg Gly Ala Val Tyr Ala Glu Asn Cys
245 250 255

Ile Leu Ala Ala Phe Ser Ile Cys Gln Lys Lys Ala Asn Leu Arg Ala
260 265 270

Gln

<210> 2
<211> 270
<212> PRT
<213> Bovine

<400> 2
Met Thr Val Asp Asp Pro Lys Gly Met Lys Asp Gln Leu Asp Gln Lys
1 5 10 15

Pro Asn Gly Lys Thr Ala Lys Gly Phe Val Ser Ser Trp Arg Trp Tyr
20 25 30

Pro Ala Ala Val Thr Leu Gly Val Leu Cys Leu Gly Leu Leu Val Thr
35 40 45

Val Ile Leu Leu Ile Leu Gln Leu Ser Gln Val Ser Asp Leu Ile Lys
50 55 60

Lys Gln Gln Ala Asn Ile Thr His Gln Glu Asp Ile Leu Glu Gly Gln
65 70 75 80

Ile Leu Ala Gln Arg Arg Ser Glu Lys Ser Ala Gln Glu Ser Gln Lys
85 90 95

Glu Leu Lys Glu Met Ile Glu Thr Leu Ala His Lys Leu Asp Glu Lys
100 105 110

Ser Lys Lys Leu Met Glu Leu His Arg Gln Asn Leu Asn Leu Gln Glu
115 120 125

Val Leu Lys Glu Ala Ala Asn Tyr Ser Gly Pro Cys Pro Gln Asp Trp
130 135 140

Leu Trp His Glu Glu Asn Cys Tyr Gln Phe Ser Ser Gly Ser Phe Asn
145 150 155 160

Trp Glu Lys Ser Gln Glu Asn Cys Leu Ser Leu Asp Ala His Leu Leu
165 170 175

Lys Ile Asn Ser Thr Asp Glu Leu Glu Phe Ile Gln Gln Met Ile Ala
180 185 190

His Ser Ser Phe Pro Phe Trp Met Gly Leu Ser Met Arg Lys Pro Asn
195 200 205

Tyr Ser Trp Leu Trp Glu Asp Gly Thr Pro Leu Thr Pro His Leu Phe
210 215 220

Arg Ile Gln Gly Ala Val Ser Arg Met Tyr Pro Ser Gly Thr Cys Ala
225 230 235 240

Tyr Ile Gln Arg Gly Thr Val Phe Ala Glu Asn Cys Ile Leu Thr Ala
245 250 255

Phe Ser Ile Cys Gln Lys Lys Ala Asn Leu Leu Arg Ala Gln
260 265 270

<210> 3
<211> 445
<212> PRT
<213> Artificial Sequence

<220>
<223> Description of Artificial Sequence:Chimeric protein consisting of extracellular region of bovine LOX-1 and Fc region of human immunoglobulin IgG1

<400> 3
Asp Leu Ile Lys Lys Gln Gln Ala Asn Ile Thr His Gln Glu Asp Ile
1 5 10 15

Leu Glu Gly Gln Ile Leu Ala Gln Arg Arg Ser Glu Lys Ser Ala Gln
20 25 30

Glu Ser Gln Lys Glu Leu Lys Glu Met Ile Glu Thr Leu Ala His Lys
35 40 45

Leu Asp Glu Lys Ser Lys Lys Leu Met Glu Leu His Arg Gln Asn Leu
50 55 60

Asn Leu Gln Glu Val Leu Lys Glu Ala Ala Asn Tyr Ser Gly Pro Cys
65 70 75 80

Pro Gln Asp Trp Leu Trp His Glu Glu Asn Cys Tyr Gln Phe Ser Ser
85 90 95

Gly Ser Phe Asn Trp Glu Lys Ser Gln Glu Asn Cys Leu Ser Leu Asp
100 105 110

Ala His Leu Leu Lys Ile Asn Ser Thr Asp Glu Leu Glu Phe Ile Gln

115 120 125

Gln Met Ile Ala His Ser Ser Phe Pro Phe Trp Met Gly Leu Ser Met
130 135 140

Arg Lys Pro Asn Tyr Ser Trp Leu Trp Glu Asp Gly Thr Pro Leu Thr
145 150 155 160

Pro His Leu Phe Arg Ile Gln Gly Ala Val Ser Arg Met Tyr Pro Ser
165 170 175

Gly Thr Cys Ala Tyr Ile Gln Arg Gly Thr Val Phe Ala Glu Asn Cys
180 185 190

Ile Leu Thr Ala Phe Ser Ile Cys Gln Lys Lys Ala Asn Leu Leu Arg
195 200 205

Ala Gln Asp Pro Glu Glu Pro Lys Ser Cys Asp Lys Thr His Thr Cys
210 215 220

Pro Pro Cys Pro Ala Pro Glu Leu Leu Gly Gly Pro Ser Val Phe Leu
225 230 235 240

Phe Pro Pro Lys Pro Lys Asp Thr Leu Met Ile Ser Arg Thr Pro Glu
245 250 255

Val Thr Cys Val Val Val Asp Val Ser His Glu Asp Pro Glu Val Lys
260 265 270

Phe Asn Trp Tyr Val Asp Gly Val Glu Val His Asn Ala Lys Thr Lys
275 280 285

Pro Arg Glu Glu Gln Tyr Asn Ser Thr Tyr Arg Val Val Ser Val Leu
290 295 300

Thr Val Leu His Gln Asp Trp Leu Asn Gly Lys Glu Tyr Lys Cys Lys
305 310 315 320

Val Ser Asn Lys Ala Leu Pro Ala Pro Ile Glu Lys Thr Ile Ser Lys
325 330 335

Ala Lys Gly Gln Pro Arg Glu Pro Gln Val Tyr Thr Leu Pro Pro Ser
340 345 350

Arg Asp Glu Leu Thr Lys Asn Gln Val Ser Leu Thr Cys Leu Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Val Leu Asp Ser Asp Gly
385 390 395 400

Ser Phe Phe Leu Tyr Ser Lys Leu Thr Val Asp Lys Ser Arg Trp Gln
405 410 415

Gln Gly Asn Val Phe Ser Cys Ser Val Met His Glu Ala Leu His Asn
420 425 430

His Tyr Thr Gln Lys Ser Leu Ser Leu Ser Pro Gly Lys
435 440 445

<210> 4
<211> 1335
<212> DNA
<213> Artificial Sequence

<220>
<223> Description of Artificial Sequence: DNA encoding

a chimeric protein consisting of extracellular region of bovine LOX-1 and Fc region of human immunoglobulin IgG1

<400> 4

```
gat ctc ata aag aaa cag caa gca aat att act cac cag gaa gat atc    48
Asp Leu Ile Lys Lys Gln Gln Ala Asn Ile Thr His Gln Glu Asp Ile
  1               5                  10                  15

ctg gag gga cag att tta gcc cag cgc cga tca gaa aaa tct gcc cag    96
Leu Glu Gly Gln Ile Leu Ala Gln Arg Arg Ser Glu Lys Ser Ala Gln
             20                  25                  30

gag tca cag aag gaa ctc aaa gaa atg ata gaa acc ctt gcc cac aag   144
Glu Ser Gln Lys Glu Leu Lys Glu Met Ile Glu Thr Leu Ala His Lys
         35                  40                  45

ctg gat gag aaa tcc aag aaa cta atg gaa ctt cac cgc cag aac ctg   192
Leu Asp Glu Lys Ser Lys Lys Leu Met Glu Leu His Arg Gln Asn Leu
     50                  55                  60

aat ctc caa gaa gtt ctg aaa gag gca gca aac tat tca ggt cct tgt   240
Asn Leu Gln Glu Val Leu Lys Glu Ala Ala Asn Tyr Ser Gly Pro Cys
 65                  70                  75                  80

ccc caa gac tgg ctc tgg cat gaa gaa aac tgt tac caa ttt tcc tct   288
Pro Gln Asp Trp Leu Trp His Glu Glu Asn Cys Tyr Gln Phe Ser Ser
                 85                  90                  95

ggc tct ttt aat tgg gaa aaa agc cag gag aac tgc ttg tct ttg gat   336
Gly Ser Phe Asn Trp Glu Lys Ser Gln Glu Asn Cys Leu Ser Leu Asp
            100                 105                 110

gcc cac ttg ctg aag att aat agc aca gat gaa ctg gaa ttc atc cag   384
Ala His Leu Leu Lys Ile Asn Ser Thr Asp Glu Leu Glu Phe Ile Gln
```

115 120 125

caa atg att gcc cat tcc agt ttc ccc ttc tgg atg ggg ttg tca atg 432
Gln Met Ile Ala His Ser Ser Phe Pro Phe Trp Met Gly Leu Ser Met
130 135 140

agg aaa ccc aat tac tcg tgg ctt tgg gaa gat ggt act cct ttg acg 480
Arg Lys Pro Asn Tyr Ser Trp Leu Trp Glu Asp Gly Thr Pro Leu Thr
145 150 155 160

ccc cac ttg ttt aga att cag gga gct gtt tcc cgt atg tat cct tca 528
Pro His Leu Phe Arg Ile Gln Gly Ala Val Ser Arg Met Tyr Pro Ser
165 170 175

ggg acc tgt gca tat att caa agg gga act gtt ttt gct gaa aac tgc 576
Gly Thr Cys Ala Tyr Ile Gln Arg Gly Thr Val Phe Ala Glu Asn Cys
180 185 190

att tta act gca ttc agt ata tgt caa aag aag gcg aat cta ttg aga 624
Ile Leu Thr Ala Phe Ser Ile Cys Gln Lys Lys Ala Asn Leu Leu Arg
195 200 205

gca cag gat ccc gag gag ccc aaa tct tgt gac aaa act cac aca tgc 672
Ala Gln Asp Pro Glu Glu Pro Lys Ser Cys Asp Lys Thr His Thr Cys
210 215 220

cca ccg tgc cca gca cct gaa ctc ctg ggg gga ccg tca gtc ttc ctc 720
Pro Pro Cys Pro Ala Pro Glu Leu Leu Gly Gly Pro Ser Val Phe Leu
225 230 235 240

ttc ccc cca aaa ccc aag gac acc ctc atg atc tcc cgg acc cct gag 768
Phe Pro Pro Lys Pro Lys Asp Thr Leu Met Ile Ser Arg Thr Pro Glu
245 250 255

gtc aca tgc gtg gtg gtg gac gtg agc cac gaa gac cct gag gtc aag 816

Val Thr Cys Val Val Val Asp Val Ser His Glu Asp Pro Glu Val Lys
260 265 270

ttc aac tgg tac gtg gac ggc gtg gag gtg cat aat gcc aag aca aag 864
Phe Asn Trp Tyr Val Asp Gly Val Glu Val His Asn Ala Lys Thr Lys
275 280 285

ccg cgg gag gag cag tac aac agc acg tac cgg gtg gtc agc gtc ctc 912
Pro Arg Glu Glu Gln Tyr Asn Ser Thr Tyr Arg Val Val Ser Val Leu
290 295 300

acc gtc ctg cac cag gac tgg ctg aat ggc aag gag tac aag tgc aag 960
Thr Val Leu His Gln Asp Trp Leu Asn Gly Lys Glu Tyr Lys Cys Lys
305 310 315 320

gtc tcc aac aaa gcc ctc cca gcc ccc atc gag aaa acc atc tcc aaa 1008
Val Ser Asn Lys Ala Leu Pro Ala Pro Ile Glu Lys Thr Ile Ser Lys
325 330 335

gcc aaa ggg cag ccc cga gaa cca cag gtg tac acc ctg ccc cca tcc 1056
Ala Lys Gly Gln Pro Arg Glu Pro Gln Val Tyr Thr Leu Pro Pro Ser
340 345 350

cgg gat gag ctg acc aag aac cag gtc agc ctg acc tgc ctg gtc aaa 1104
Arg Asp Glu Leu Thr Lys Asn Gln Val Ser Leu Thr Cys Leu Val Lys
355 360 365

ggc ttc tat ccc agc gac atc gcc gtg gag tgg gag agc aat ggg cag 1152
Gly Phe Tyr Pro Ser Asp Ile Ala Val Glu Trp Glu Ser Asn Gly Gln
370 375 380

ccg gag aac aac tac aag acc acg cct ccc gtg ctg gac tcc gac ggc 1200
Pro Glu Asn Asn Tyr Lys Thr Thr Pro Pro Val Leu Asp Ser Asp Gly
385 390 395 400

tcc ttc ttc ctc tac agc aag ctc acc gtg gac aag agc agg tgg cag 1248
Ser Phe Phe Leu Tyr Ser Lys Leu Thr Val Asp Lys Ser Arg Trp Gln
405 410 415

cag ggg aac gtc ttc tca tgc tcc gtg atg cat gag gct ctg cac aac 1296
Gln Gly Asn Val Phe Ser Cys Ser Val Met His Glu Ala Leu His Asn
420 425 430

cac tac acg cag aag agc ctc tcc ctg tct ccg ggt aaa 1335
His Tyr Thr Gln Lys Ser Leu Ser Leu Ser Pro Gly Lys
435 440 445

<210> 5
<211> 1318
<212> DNA
<213> Homo sapiens

<220>
<221> 5'UTR
<222> (1)..(126)

<220>
<221> CDS
<222> (127)..(948)

<220>
<221> 3'UTR
<222> (949)..(1318)

<400> 5
ggggccgcac tagtgattct ggttcggccc acctctgaag gttccagaat cgatagtgaa 60
ttcgtgattt tagtttgttg aagttcgtga ctgcttcact ctctcattct tagcttgaat 120
ttggaa atg act ttt gat gac cta aag atc cag act gtg aag gac cag 168
Met Thr Phe Asp Asp Leu Lys Ile Gln Thr Val Lys Asp Gln

```
            1               5                  10
cct gat gag aag tca aat gga aaa aaa gct aaa ggt ctt cag ttt ctt   216
Pro Asp Glu Lys Ser Asn Gly Lys Lys Ala Lys Gly Leu Gln Phe Leu
 15                  20                  25                  30

tac tct cca tgg tgg tgc ctg gct gct gcg act cta ggg gtc ctt tgc   264
Tyr Ser Pro Trp Trp Cys Leu Ala Ala Ala Thr Leu Gly Val Leu Cys
                     35                  40                  45

ctg gga tta gta gtg acc att atg gtg ctg ggc atg caa tta tcc cag   312
Leu Gly Leu Val Val Thr Leu Met Val Leu Gly Met Gln Leu Ser Gln
                 50                  55                  60

gtg tct gac ctc cta aca caa gag caa gca aac cta act cac cag aaa   360
Val Ser Asp Leu Leu Thr Gln Glu Gln Ala Asn Leu Thr His Gln Lys
             65                  70                  75

aag aaa ctg gag gga cag atc tca gcc cgg caa caa gca gaa gaa gct   408
Lys Lys Leu Glu Gly Gln Ile Ser Ala Arg Gln Gln Ala Glu Glu Ala
     80                  85                  90

tca cag gag tca gaa aac gaa ctc aag gaa atg ata gaa acc ctt gct   456
Ser Gln Glu Ser Glu Asn Glu Leu Lys Glu Met Ile Glu Thr Leu Ala
 95                 100                 105                 110

cgg aag ctg aat gag aaa tcc aaa gag caa atg gaa ctt cac cac cag   504
Arg Lys Leu Asn Glu Lys Ser Lys Glu Gln Met Glu Leu His His Gln
                115                 120                 125

aat ctg aat ctc caa gaa aca ctg aag aga gta gca aat tgt tca gct   552
Asn Leu Asn Leu Gln Glu Thr Leu Lys Arg Val Ala Asn Cys Ser Ala
            130                 135                 140

cct tgt ccg caa gac tgg atc tgg cat gga gaa aac tgt tac cta ttt   600
Pro Cys Pro Gln Asp Trp Ile Trp His Gly Glu Asn Cys Tyr Leu Phe
```

145 150 155

tcc tcg ggc tca ttt aac tgg gaa aag agc caa gag aag tgc ttg tct 648
Ser Ser Gly Ser Phe Asn Trp Glu Lys Ser Gln Glu Lys Cys Leu Ser
160 165 170

ttg gat gcc aag ttg ctg aaa att aat agc aca gct gat ctg gac ttc 696
Leu Asp Ala Lys Leu Leu Lys Ile Asn Ser Thr Ala Asp Leu Asp Phe
175 180 185 190

atc cag caa gca att tcc tat tcc agt ttt cca ttc tgg atg ggg ctg 744
Ile Gln Gln Ala Ile Ser Tyr Ser Ser Phe Pro Phe Trp Met Gly Leu
195 200 205

tct cgg agg aac ccc agc tac cca tgg ctc tgg gag gac ggt tct cct 792
Ser Arg Arg Asn Pro Ser Tyr Pro Trp Leu Trp Glu Asp Gly Ser Pro
210 215 220

ttg atg ccc cac tta ttt aga gtc cga ggc gct gtc tcc cag aca tac 840
Leu Met Pro His Leu Phe Arg Val Arg Gly Ala Val Ser Gln Thr Tyr
225 230 235

cct tca ggt acc tgt gca tat ata caa cga gga gct gtt tat gcg gaa 888
Pro Ser Gly Thr Cys Ala Tyr Ile Gln Arg Gly Ala Val Tyr Ala Glu
240 245 250

aac tgc att tta gct gcc ttc agt ata tgt cag aag aag gca aac cta 936
Asn Cys Ile Leu Ala Ala Phe Ser Ile Cys Gln Lys Lys Ala Asn Leu
255 260 265 270

aga gca cag tga atttgaaggc tctggaagaa aagaaaaaag tctttgagtt 988
Arg Ala Gln

ttattctgga atttaagcta ttctttgtca cttgggtgcc aaacatgaga gcccagaaaa 1048
ctgtcattta gctggctgca gaactccttt gcagaaactg gggttccagg tgcctggcac 1188

ctttatgtca acatttttga ttctagctat ctgtattatt tcacctagct tgtcccaagc 1168
ttccctgcca gcctgaagtc cattttcccc tttttatttt aaaatttgac tcctcttcaa 1228
gcttgaaaac cctctgaact cagtcttctt tacctcatta tcaccttccc ctcacactcc 1288
taaaattgca tgaaagacag accggaattc 1318

<210> 6
<211> 1897
<212> DNA
<213> Bovine

<220>
<221> 5'UTR
<222> (1)..(34)

<220>
<221> CDS
<222> (35)..(847)

<220>
<221> 3'UTR
<222> (848)..(1897)

<220>
<221> polyA_signal
<222> (1859)..(1864)

<220>
<221> polyA_site
<222> (1880)..(1897)

<400> 6
gcttcactct ctcattcttg gaatacattt gaaa atg act gtt gat gac ccc 52
Met thr Val Asp Asp Pro
1 5

```
aag ggt atg aaa gat caa ctt gat cag aag cca aat ggc aag aca gca    100
Lys Gly Met Lys Asp Gln Leu Asp Gln Lys Pro Asn Gly Lys Thr Ala
                10                  15                  20

aaa ggt ttt gtt tcc tct tgg agg tgg tac cct gct gct gtg act cta    148
Lys Gly Phe Val Ser Ser Trp Arg Trp Tyr Pro Ala Ala Val Thr Leu
            25                  30                  35

ggg gtc ctt tgt ctg gga tta ctg gtg act gtt ata ttg ttg ata ctg    196
Gly Val Leu Cys Leu Gly Leu Leu Val Thr Val Ile Leu Leu Ile Leu
        40                  45                  50

caa tta tcc cag gtc tct gat ctc ata aag aaa cag caa gca aat att    244
Gln Leu Ser Gln Val Ser Asp Leu Ile Lys Lys Gln Gln Ala Asn Ile
    55                  60                  65                  70

act cac cag gaa gat atc ctg gag gga cag att tta gcc cag cgc cga    292
Thr His Gln Glu Asp Ile Leu Glu Gly Gln Ile Leu Ala Gln Arg Arg
                    75                  80                  85

tca gaa aaa tct gcc cag gag tca cag aag gaa ctc aaa gaa atg ata    340
Ser Glu Lys Ser Ala Gln Glu Ser Gln Lys Glu Leu Lys Glu Met Ile
                90                  95                  100

gaa acc ctt gcc cac aag ctg gat gag aaa tcc aag aaa cta atg gaa    388
Glu thr Leu Ala His Lys Leu Asp Glu Lys Ser Lys Lys Leu Met Glu
            105                 110                 115

ctt cac cgc cag aac ctg aat ctc caa gaa gtt ctg aaa gag gca gca    436
Leu His Arg Gln Asn Leu Asn Leu Gln Glu Val Leu Lys Glu Ala Ala
        120                 125                 130

aac tat tca ggt cct tgt ccc caa gac tgg ctc tgg cat gaa gaa aac    484
Asn Tyr Ser Gly Pro Cys Pro Gln Asp Trp Leu Trp His Glu Glu Asn
```

135 140 145 150

tgt tac caa ttt tcc tct ggc tct ttt aat tgg gaa aaa agc cag gag 532
Cys Tyr Gln Phe Ser Ser Gly Ser Phe Asn Trp Glu Lys Ser Gln Glu
155 160 165

aac tgc ttg tct ttg gat gcc cac ttg ctg aag att aat agc aca gat 580
Asn Cys Leu Ser Leu Asp Ala His Leu Leu Lys Ile Asn Ser Thr Asp
170 175 180

gaa ctg gaa ttc atc cag caa atg att gcc cat tcc agt ttc ccc ttc 628
Glu Leu Glu Phe Ile Gln Gln Met Ile Ala His Ser Ser Phe Pro Phe
185 190 195

tgg atg ggg ttg tca atg agg aaa ccc aat tac tcg tgg ctt tgg gaa 676
Trp Met Gly Leu Ser Met Arg Lys Pro Asn Tyr Ser Trp Leu Trp Glu
200 205 210

gat ggt act cct ttg acg ccc cac ttg ttt aga att cag gga gct gtt 724
Asp Gly Thr Pro Leu Thr Pro His Leu Phe Arg Ile Gln Gly Ala Val
215 220 225 230

tcc cgt atg tat cct tca ggg acc tgt gca tat att caa agg gga act 772
Ser Arg Met Tyr Pro Ser Gly Thr Cys Ala Tyr Ile Gln Arg Gly Thr
235 240 245

gtt ttt gct gaa aac tgc att tta act gca ttc agt ata tgt caa aag 820
Val Phe Ala Glu Asn Cys Ile Leu Thr Ala Phe Ser Ile Cys Gln Lys
250 255 260

aag gcg aat cta ttg aga gca cag tga atttgaagga tctggaggaa 867
Lys Ala Asn Leu Leu Arg Ala Gln
265 270

aagaaggaaa cctttgaatt ctcttctgga atttaagcta tacttcatca cttagatgta 927

```
aaccattaga gcccagggaa atgcctgcta ctggttgagt gcagaactcc ttagcagaga 987
ctggcccagc tgcctggcac cttgatagca aaagttgcaa ttccctctgt atatttttcc 1047
ctaacttgtt ccaagtcctc ccctgcagga cttcagagaa gtcaattttt ctgtttccat 1107
tgtttctaag aacttgttgc ctaactcaag gtcacagcat ttttctcact tttgtcctat 1167
gctttcttct aggcattgta gagttttaga ttttacatgg aaatctagaa cttattttag 1227
attaatttct aagtgatata tggatgtatg gaagttttct gtttgttttt tgcttgtgag 1287
tattcaattg tttttgcaac atttgctgaa aagactattc ttccttcact acattgcctt 1347
tgcactgttg tcaacaatta tccatacatg cctggctcta tttctggatt ttctattcct 1407
ttccatttat ttatttatta ttcttggctt acaacatcac catgatattt tgaattctat 1467
ggttctttaa tatatcttgg aatcacatgg tagtagttat tcattgttgt tctttttag 1527
agttgtttgg ttaatctatg cttttgtatt tctgtcttaa attggcttgt ccatttctaa 1587
aaaaacttga aattttgaat tgcactgaat ccatacataa atttagggaa aattgaattc 1647
ttaaaaatac tgatttgttc aactcatgaa aaaggtgtat tgctctattt aggtattcct 1707
tattttcttt aagcaatgct ttttaatgtt ctttgtgtag atattgttag attatcatca 1767
tgtatttcac attatttatg ctactgtaga tagtattgtt atcatttgtt gttcttattt 1827
tcaaagtctt ctgctagtat gtagaattat aataaagttt gatattaata ttaaaaaaaa 1887
aaaaaaaaaa                                                        1897
```

<210> 7
<211> 1459
<212> DNA
<213> Artificial Sequence

<220>
<223> Description of Artificial Sequence: Vector DNA of pCd5lnegl containing genomic DNA comprising exons encoding a Fc region of human immunoglobulin IgG1

<400> 7

```
ctcgagatcc attgtgctct aaaggagata cccggccaga caccctcacc tgcggtgccc 60
agctgcccag gctgaggcaa gagaaggcca gaaacc atg ccc atg ggg tct ctg   114
                                        Met Pro Met Gly Ser Leu
                                         1               5
```

caa ccg ctg gcc acc ttg tac ctg ctg ggg atg ctg gtc gct tcc gtg 162
Gln Pro Leu Ala Thr Leu Tyr Leu Leu Gly Met Leu Val Ala Ser Val
10 15 20

cta gcg gat ccc gag ggtgagtact aagcttcagc gctcctgcct ggacgcatcc 217
Leu Ala Asp Pro Glu
25

cggctatgca gccccagtcc agggcagcaa ggcaggcccc gtctgcctct tcacccggag 277
cctctgcccg ccccactcat gctcagggag agggtcttct ggctttttcc caggctctgg 337
gcaggcacag gctaggtgcc cctaacccag gccctgcaca caaaggggca ggtgctgggc 397
tcagacctgc caagagccat atccgggagg accctgcccc tgacctaagc ccaccccaaa 457
ggccaaactc tccactccct cagctcggac accttctctc ctcccagatt ccagtaactc 517
ccaatcttct ctctgca gag ccc aaa tct tgt gac aaa act cac aca tgc 567
Glu Pro Lys Ser Cys Asp Lys Thr His Thr Cys
30 35

cca ccg tgc cca ggtaagccag cccaggcctc gccctccagc tcaaggcggg 619
Pro Pro Cys Pro
40

acaggtgccc tagagtagcc tgcatccagg gacaggcccc agccgggtgc tgacacgtcc 679
acctccatct cttcctca gca cct gaa ctc ctg ggg gga ccg tca gtc ttc 730
Ala Pro Glu Leu Leu Gly Gly Pro Ser Val Phe
45 50

ctc ttc ccc cca aaa ccc aag gac acc ctc atg atc tcc cgg acc cct 778
Leu Phe Pro Pro Lys Pro Lys Asp Thr Leu Met Ile Ser Arg Thr Pro
55 60 65

gag gtc aca tgc gtg gtg gtg gac gtg agc cac gaa gac cct gag gtc 826
Glu Val Thr Cys Val Val Val Asp Val Ser His Glu Asp Pro Glu Val
70 75 80 85

aag ttc aac tgg tac gtg gac ggc gtg gag gtg cat aat gcc aag aca 874

Lys Phe Asn Trp Tyr Val Asp Gly Val Glu Val His Asn Ala Lys Thr
90 95 100

aag ccg cgg gag gag cag tac aac agc acg tac cgg gtg gtc agc gtc 922
Lys Pro Arg Glu Glu Gln Tyr Asn Ser Thr Tyr Arg Val Val Ser Val
105 110 115

ctc acc gtc ctg cac cag gac tgg ctg aat ggc aag gag tac aag tgc 970
Leu Thr Val Leu His Gln Asp Trp Leu Asn Gly Lys Glu Tyr Lys Cys
120 125 130

aag gtc tcc aac aaa gcc ctc cca gcc ccc atc gag aaa acc atc tcc 1018
Lys Val Ser Asn Lys Ala Leu Pro Ala Pro Ile Glu Lys Thr Ile Ser
135 140 145

aaa gcc aaa ggtgggaccc gtggggtgcg agggccacat ggacagaggc 1067
Lys Ala Lys
150

cggctcggcc caccctctgc cctgagagtg accgctgtac caacctctgt cctaca ggg 1126
Gly

cag ccc cga gaa cca cag gtg tac acc ctg ccc cca tcc cgg gat gag 1174
Gln Pro Arg Glu Pro Gln Val Tyr Thr Leu Pro Pro Ser Arg Asp Glu
155 160 165

ctg acc aag aac cag gtc agc ctg acc tgc ctg gtc aaa ggc ttc tat 1222
Leu Thr Lys Asn Gln Val Ser Leu Thr Cys Leu Val Lys Gly Phe Tyr
170 175 180 185

ccc agc gac atc gcc gtg gag tgg gag agc aat ggg cag ccg gag aac 1270
Pro Ser Asp Ile Ala Val Glu Trp Glu Ser Asn Gly Gln Pro Glu Asn
190 195 200

aac tac aag acc acg cct ccc gtg ctg gac tcc gac ggc tcc ttc ttc 1318

Asn Tyr Lys Thr Thr Pro Pro Val Leu Asp Ser Asp Gly Ser Phe Phe
205 210 215

ctc tac agc aag ctc acc gtg gac aag agc agg tgg cag cag ggg aac 1366
Leu Tyr Ser Lys Leu Thr Val Asp Lys Ser Arg Trp Gln Gln Gly Asn
220 225 230

gtc ttc tca tgc tcc gtg atg cat gag gct ctg cac aac cac tac acg 1414
Val Phe Ser Cys Ser Val Met His Glu Ala Leu His Asn His Tyr Thr
235 240 245

cag aag agc ctc tcc ctg tct ccg ggt aaa tga gtgcgacggc cg 1459
Gln Lys Ser Leu Ser Leu Ser Pro Gly Lys
250 255

<210> 8
<211> 27
<212> DNA
<213> Artificial Sequence

<220>
<223> Description of Artificial Sequence:Artificially synthesized primer sequence

<220>
<221> primer_bind
<222> (1)..(27)

<400> 8
ggggatcctg atctcataaa gaaacag 27

<210> 9
<211> 28

<212> DNA
<213> Artificial Sequence

<220>
<223> Description of Artificial Sequence:Artificially synthesized primer sequence

<220>
<221> primer_bind
<222> (1)..(28)

<400> 9
gcggatcctg tgctctcaat agattcgc 28

<210> 10
<211> 1921
<212> DNA
<213> Artificial Sequence

<220>
<223> Description of Artificial Sequence: DNA consisting of a DNA encoding an extracellular region of bovine LOX-1 and genomic DNA comprising exons encoding a Fc region of human immunoglobulin IgG1

<400> 10
```
gat ctc ata aag aaa cag caa gca aat att act cac cag gaa gat atc   48
Asp Leu Ile Lys Lys Gln Gln Ala Asn Ile Thr His Gln Glu Asp Ile
  1               5                  10                  15

ctg gag gga cag att tta gcc cag cgc cga tca gaa aaa tct gcc cag   96
Leu Glu Gly Gln Ile Leu Ala Gln Arg Arg Ser Glu Lys Ser Ala Gln
             20                  25                  30
```

gag tca cag aag gaa ctc aaa gaa atg ata gaa acc ctt gcc cac aag 144
Glu Ser Gln Lys Glu Leu Lys Glu Met Ile Glu Thr Leu Ala His Lys
35 40 45

ctg gat gag aaa tcc aag aaa cta atg gaa ctt cac cgc cag aac ctg 192
Leu Asp Glu Lys Ser Lys Lys Leu Met Glu Leu His Arg Gln Asn Leu
50 55 60

aat ctc caa gaa gtt ctg aaa gag gca gca aac tat tca ggt cct tgt 240
Asn Leu Gln Glu Val Leu Lys Glu Ala Ala Asn Tyr Ser Gly Pro Cys
65 70 75 80

ccc caa gac tgg ctc tgg cat gaa gaa aac tgt tac caa ttt tcc tct 288
Pro Gln Asp Trp Leu Trp His Glu Glu Asn Cys Tyr Gln Phe Ser Ser
85 90 95

ggc tct ttt aat tgg gaa aaa agc cag gag aac tgc ttg tct ttg gat 336
Gly Ser Phe Asn Trp Glu Lys Ser Gln Glu Asn Cys Leu Ser Leu Asp
100 105 110

gcc cac ttg ctg aag att aat agc aca gat gaa ctg gaa ttc atc cag 384
Ala His Leu Leu Lys Ile Asn Ser Thr Asp Glu Leu Glu Phe Ile Gln
115 120 125

caa atg att gcc cat tcc agt ttc ccc ttc tgg atg ggg ttg tca atg 432
Gln Met Ile Ala His Ser Ser Phe Pro Phe Trp Met Gly Leu Ser Met
130 135 140

agg aaa ccc aat tac tcg tgg ctt tgg gaa gat ggt act cct ttg acg 480
Arg Lys Pro Asn Tyr Ser Trp Leu Trp Glu Asp Gly Thr Pro Leu Thr
145 150 155 160

ccc cac ttg ttt aga att cag gga gct gtt tcc cgt atg tat cct tca 528
Pro His Leu Phe Arg Ile Gln Gly Ala Val Ser Arg Met Tyr Pro Ser
165 170 175

```
ggg acc tgt gca tat att caa agg gga act gtt ttt gct gaa aac tgc    576
Gly Thr Cys Ala Tyr Ile Gln Arg Gly Thr Val Phe Ala Glu Asn Cys
            180                 185                 190

att tta act gca ttc agt ata tgt caa aag aag gcg aat cta ttg aga    624
Ile Leu Thr Ala Phe Ser Ile Cys Gln Lys Lys Ala Asn Leu Leu Arg
        195                 200                 205

gca cag gat ccc gag ggtgagtact aagcttcagc gctcctgcct ggacgcatcc    679
Ala Gln Asp Pro Glu
    210

cggctatgca gccccagtcc agggcagcaa ggcaggcccc gtctgcctct tcacccggag 739
cctctgcccg ccccactcat gctcagggag agggtcttct ggctttttcc caggctctgg 799
gcaggcacag gctaggtgcc cctaacccag gccctgcaca caaaggggca ggtgctgggc 859
tcagacctgc caagagccat atccgggagg accctgcccc tgacctaagc ccaccccaaa 919
ggccaaactc tccactccct cagctcggac accttctctc ctcccagatt ccagtaactc 979
ccaatcttct ctctgca gag ccc aaa tct tgt gac aaa act cac aca tgc    1029
                   Glu Pro Lys Ser Cys Asp Lys Thr His Thr Cys
                           215                 220

cca ccg tgc cca ggtaagccag cccaggcctc gccctccagc tcaaggcggg        1081
Pro Pro Cys Pro
225

acaggtgccc tagagtagcc tgcatccagg gacaggcccc agccgggtgc tgacacgtcc 1141
acctccatct cttcctca gca cct gaa ctc ctg ggg gga ccg tca gtc ttc   1192
                    Ala Pro Glu Leu Leu Gly Gly Pro Ser Val Phe
                        230                 235

ctc ttc ccc cca aaa ccc aag gac acc ctc atg atc tcc cgg acc cct    1240
Leu Phe Pro Pro Lys Pro Lys Asp Thr Leu Met Ile Ser Arg Thr Pro
240                 245                 250                 255
```

gag gtc aca tgc gtg gtg gtg gac gtg agc cac gaa gac cct gag gtc 1288
Glu Val Thr Cys Val Val Val Asp Val Ser His Glu Asp Pro Glu Val
260 265 270

aag ttc aac tgg tac gtg gac ggc gtg gag gtg cat aat gcc aag aca 1336
Lys Phe Asn Trp Tyr Val Asp Gly Val Glu Val His Asn Ala Lys Thr
275 280 285

aag ccg cgg gag gag cag tac aac agc acg tac cgg gtg gtc agc gtc 1384
Lys Pro Arg Glu Glu Gln Tyr Asn Ser Thr Tyr Arg Val Val Ser Val
290 295 300

ctc acc gtc ctg cac cag gac tgg ctg aat ggc aag gag tac aag tgc 1432
Leu Thr Val Leu His Gln Asp Trp Leu Asn Gly Lys Glu Tyr Lys Cys
305 310 315

aag gtc tcc aac aaa gcc ctc cca gcc ccc atc gag aaa acc atc tcc 1480
Lys Val Ser Asn Lys Ala Leu Pro Ala Pro Ile Glu Lys Thr Ile Ser
320 325 330 335

aaa gcc aaa ggtgggaccc gtggggtgcg agggccacat ggacagaggc 1529
Lys Ala Lys

cggctcggcc caccctctgc cctgagagtg accgctgtac caacctctgt cctaca 1585
ggg cag ccc cga gaa cca cag gtg tac acc ctg ccc cca tcc cgg gat 1633
Gly Gln Pro Arg Glu Pro Gln Val Tyr Thr Leu Pro Pro Ser Arg Asp
340 345 350

gag ctg acc aag aac cag gtc agc ctg acc tgc ctg gtc aaa ggc ttc 1681
Glu Leu Thr Lys Asn Gln Val Ser Leu Thr Cys Leu Val Lys Gly Phe
355 360 365 370

tat ccc agc gac atc gcc gtg gag tgg gag agc aat ggg cag ccg gag 1729
Tyr Pro Ser Asp Ile Ala Val Glu Trp Glu Ser Asn Gly Gln Pro Glu
375 380 385

aac aac tac aag acc acg cct ccc gtg ctg gac tcc gac ggc tcc ttc 1777
Asn Asn Tyr Lys Thr Thr Pro Pro Val Leu Asp Ser Asp Gly Ser Phe
390 395 400

ttc ctc tac agc aag ctc acc gtg gac aag agc agg tgg cag cag ggg 1825
Phe Leu Tyr Ser Lys Leu Thr Val Asp Lys Ser Arg Trp Gln Gln Gly
405 410 415

aac gtc ttc tca tgc tcc gtg atg cat gag gct ctg cac aac cac tac 1873
Asn Val Phe Ser Cys Ser Val Met His Glu Ala Leu His Asn His Tyr
420 425 430

acg cag aag agc ctc tcc ctg tct ccg ggt aaa tga gtgcgacggc cg 1921
Thr Gln Lys Ser Leu Ser Leu Ser Pro Gly Lys
435 440 445